# EUROPÄISCHE PATENTSCHRIFT

(11) **EP 2 240 882 B1**
(45) Veröffentlichungstag und Bekanntmachung des Hinweises auf die Patenterteilung: **12.12.2012**
(21) Anmeldenummer: 09700565.6
(22) Anmeldetag: 09.01.2009
(51) Int. Cl.: G06K 9/00, G07C 9/00

(54) **BIOMETRISCHE SICHERUNGSVORRICHTUNG**
BIOMETRIC SECURITY DEVICE
DISPOSITIF DE PROTECTION BIOMÉTRIQUE

(30) Priorität: 09.01.2008 AT 302008
(43) Veröffentlichungstag der Anmeldung: 20.10.2010
(73) Patentinhaber: ASMAG-Holding GmbH, 4645 Grünau im Almtal (AT)
(72) Erfinder: SCHRÖTER, Klaus, 10707 Berlin (DE)
(74) Vertreter: Ofner, Clemens
(86) Internationale Anmeldenummer: PCT/AT2009/000004
(87) Internationale Veröffentlichungsnummer: WO 2009/086576

(56) Entgegenhaltungen:
- EP-A- 1 811 452
- WO-A-03/021523
- WO-A-2006/026794
- US-A- 6 078 265
- US-A1- 2004 222 420
- US-A1- 2007 098 227
- ZIMMERMAN T G ET AL: "APPLYING ELECTRIC FIELD SENSING TO HUMAN-COMPUTER INTERFACES", HUMAN FACTORS IN COMPUTING SYSTEMS. CHI '95 CONFERENCE PROCEEDINGS. DENVER, MAY 7 - 11, 1995; [CONFERENCE ON HUMAN FACTORS IN COMPUTING SYSTEMS], NEW YORK, ACM, US, 7 May 1995 (1995-05-07), pages 280-287, XP000538457, ISBN: 978-0-201-84705-5

## Beschreibung

Die Erfindung betrifft eine Sicherungsvorrichtung zum Schutz einer technischen Vorrichtung gegen unbefugte Benützung bzw. gegen unbefugten Zugriff. Die Sicherungsvorrichtung umfasst zumindest eine Authentifizierungsvorrichtung und eine Verriegelungsvorrichtung wobei die Authentifizierungsvorrichtung zumindest einen Sensor und ein Auswerte- und Vergleichsmodul umfasst und wobei zwischen der Verriegelungsvorrichtung und der Authentifizierungsvorrichtung eine Kommunikationsverbindung besteht.

Technische Geräte bzw. technische Vorrichtungen bspw. Sicherheitsbereiche, Sicherheitstüren, Datenverarbeitungseinrichtungen und Feuerwaffen bedürfen Vorkehrungen, um sicherzustellen, das nur befugte bzw. berechtigte Personen die Vorrichtung bestimmungsgemäß benützen können bzw. dass nur diesen Personen ein Zutritt gestattet wird. Da mit derartigen Vorrichtungen zumeist sicherheitskritische bzw. geheim zu haltende Informationen verwaltet und/oder aufbewahrt werden, oder durch die Benutzung der Vorrichtung für den Benutzer selbst bzw. für die Umgebung und andere Personen eine besondere Gefahr besteht, muss eine Sicherungsvorrichtung eine besonders hohe Zuverlässigkeit hinsichtlich der eindeutigen Erkennung der befugten Person bieten. Insbesondere wird von einer derartigen Sicherungsvorrichtung gefordert, dass zur Identifikation des Benutzers solche Merkmale verwendet werden, die eindeutig sind und sich nicht bzw. nur äußerst schwer falschen oder manipulieren lassen.

Aus dem Stand der Technik sind mehrere Verfahren bekannt, die zur Identifikation einer befugten Person biometrische Daten verwenden.

Die EP 0 691 822 B 1 offenbart eine Anordnung zur Erfassung biometrischer Merkmale des Handrückens einer Person. Der Benutzer umfasst dabei fest einen Handgriff, wodurch an der Handrückenoberfläche die Venen- und Sehnenstruktur deutlich sichtbar wird. Die Handrückenoberfläche wird von einem optischen Aufnahmesystem, insbesondere einer Kamera, aufgenommen und daraus, mittels eines bildgebenden Verfahrens, die charakteristischen Erhebungen errechnet. Dieses charakteristische Muster wird als Referenzwert hinterlegt und dient bei einer späteren Verifikationsanfrage als Basis für den Vergleich auf Übereinstimmung. Durch bildgebende Verfahren und Differenzbildung wird aus dem zur Authentifikation vorgelegten Handrückenmuster und einem Referenzmuster ein Differenzbild ermittelt, in dem alle Abweichungen zwischen Referenzmuster und neu vorgelegtem Muster dargestellt sind. Bei größtmöglicher Übereinstimmung ist somit eine zuverlässige Authentifikation des Benutzers gegeben.

Auch die WO 88/04153 offenbart eine Vorrichtung, bei der die Oberflächenstruktur des Handrückens, insbesondere wiederum die Venenstruktur, zur eindeutigen Identifikation eines Benutzers verwendet wird. Das Dokument offenbart, dass der Benutzer die eine Hand in eine Vorrichtung einlegt und eine Kamera das Oberflächenbild des Handrückens aufnimmt. Mittels bildgebender Verfahren wird daraus wiederum die Oberflächenstruktur bzw. die Venenstruktur berechnet; eine Korrelationsfunktion zeigt die Übereinstimmung mit einem Referenzmuster an.

Die US 6,799,726 B2 offenbart eine Sicherungsvorrichtung in der Form einer Armbanduhr. Ein biometrischer Sensor ist in eine Armbanduhr integriert und zur Aufnahme der Venenstruktur ausgebildet. Die Armbanduhr versorgt den Sensor mit elektrischer Energie, bereitet die erfassten biometrischen Rohdaten entsprechend auf und überträgt ein biometrisches Identifikationsmerkmal über eine drahtlose Kommunikationsverbindung an eine Auswertevorrichtung.

Dokument US 6,078,265 A offenbart ein schlüsselbasiertes Sicherheitssystem, welches einen Fingerabdruck eines autorisierten Benutzers erfasst und durch Vergleichen mit hinterlegten Referenz-Fingerabdrücken ein Freigabesignal generiert und dadurch die Zündung eines Kraftfahrzeuges freigibt. Der Schlüssel hat elektrische Kontakte um den Fingerabdruckscanner mit Energie zu versorgen bzw. um eine Übertragung des erfassten Fingerabdrucks an eine Auswerteschaltung zu ermöglichen. Neben der Erfassung und Vergleich eines Fingerabdrucks einer autorisierten Person können ferner auch jene Fingerabdrücke erfasst werden, die zu einer fehlerhaften Authentifizierung führten, um später zu Strafverfolgungszwecken verwendet zu werden. Eine Umgehung der Sicherheitsfunktion, beispielsweise durch Kurzschließen des Startschalters, ist nicht möglich, da von der Fingerabdruckauswertevorrichtung die Freigabe der Zündung des Fahrzeuges nicht gegeben wird. Auch kann für autorisierte Benutzer ein Profil hinterlegt werden um beispielsweise Sitz- und Lenkradposition automatisch nach der Erkennung der Person anzupassen. Zum Auslesen des Fingerabdrucks ist vorgesehen, dass der Schlüssel zwischen Daumen und Vorfinger gehalten wird und dabei von der Fingerabdruckerfassungsvorrichtung dieser Fingerabdruck erfasst wird und von einer Auswertevorrichtung mit einem oder mehreren hinterlegten Fingerabdrücken von autorisierten Benutzern verglichen wird und darauf basierend, die Sicherheitsfunktionen des zu sichernden Systems kontrolliert werden.

Dokument US 2004/0222420 A1 offenbart einen Bilderfassungsdetektor, welcher eine Lichtquelle zur Aussendung von Licht eines bestimmten Signals aufweist. Ferner ist ein Dünnfilm-Fototransistor vorhanden, der einen Fotostrom in Abhängigkeit von der Intensität des externen Lichts generiert, wobei dieser Fotostrom einen Speicherkondensator lädt, welcher über einen Dünnfilm-Schalttransistor ausgelesen und an eine externe Auswerteeinheit übermittelt wird. Der optische Sensor ist insbesondere derart ausgebildet, dass er unterscheiden kann, ob das einfallende Licht von einem lebenden Objekt, beispielsweise einem menschlichen Finger, oder von einer Fotografie bzw. Fotokopie stammt. Dazu ist ein leitfähiges Erfassungsmuster vorgesehen, welches erste und zweite Elektroden aufweist. Durch Anlegen eines elektrischen Signals an eine der Elektroden, kann durch Auswertung des von der anderen Elektrode erfassten Signals festgestellt werden, ob das einfallende Licht von einem menschlichen Objekt stamm oder nicht.

Dokument EP 1 811 452 A1 offenbart ein Informationsverarbeitungssystem und Informationsverarbeitungsvorrichtungen, wobei eine erste Informationsverarbeitungsvorrichtung an einer vorbestimmten Position eines lebenden Körpers angeordnet wird und eine zweite Informationsverarbeitungsvorrichtung ein Bilderfassungsmittel aufweist, welche ein Abbild eines lebendigen Körperteils erfasst, welcher im Nahbereich der zweiten Informationserfassungsvorrichtung bzw. der Bilderfassungsvorrichtung angeordnet wird. Von einem Benutzer wird ein Abdruck der Blutkapillaren erfasst und in jedem einzelnen Kartenterminal, beispielsweise einer Chipkarte gespeichert. Zur Identifizierung bringt ein Benutzer das Kartenterminal sowie den zuvor erfassten Körperteil in den Nahbereich einer Erfassungsvorrichtung, wobei ein Kapillarmuster erfasst und mit dem im Kartenterminal gespeicherten Kapillarmuster verglichen wird. Zum Auslesen umfasst das Kartenterminal eine Antennenspule, ferner ist eine Terminalkennung und ein hinterlegtes Kapillarmuster vorhanden.

Das Dokument US 2007/0098227 A1 offenbart einen optischen Biosensor, der in der Lage ist, einen Fingerabdruck bzw. ein Venenmuster mit einer großen Lichterfassungseffizienz aufzunehmen. Das von einer Lichtquelle ausgesandte Licht weist dazu eine sehr hohe Direktivität auf, wodurch sich die Intensität des vom biologischen Objekt zurückgeworfenen Lichts erhöht. Zur Erreichung dieser hohen Richtwirkung ist eine Jalousie vorgesehen, um das von der Lichtquelle emittierte Licht auf die Probe zu lenken. Die Richtwirkung der Jalousie ist dabei derart ausgebildet, dass das Licht unter einem Winkel auf das zu erfassende Objekt einfällt, bei welchem ein möglichst hoher Kontrast der Fingerminutien bzw. des Venenmusters erreicht wird.

Dokument WO 03/021523 A1 offenbart eine tragbare drahtlose Transaktionsfreigabevorrichtung mit biometrischer Benutzervalidierung. Die biometrische Datenlesevorrichtung kann benutzerbezogene biometrische Daten erfassen und diese an eine Benutzeridentifizierungsvorrichtung übermitteln, welche daraufhin die Benutzerautorisierung für den angeforderten Dienst freigeben kann. Die Verwendung von Smartcards für persönliche Transaktionen, beispielsweise Käufe oder andere Transaktionen, welche die Validierung der Benutzeridentität erfordern, haben sich verbreitet durchgesetzt. Derartige Smartcards verwenden eine oder mehrere kurzreichweitige Funksysteme, beispielsweise Bluetooth. Bei Anbringung der Smartcard in die Näher einer Antenne lässt sich drahtlos der Inhalt ausgelesen. Das Dokument offenbart ferner eine Uhr als Authentifizierungsvorrichtung, bei der eine Position aus einem GPS-Empfänger ermittelt und angezeigt wird und welche ferner einen Fingerprintscanner aufweist. Der Benutzer legt einen entsprechenden Finger auf den Fingerabdruckscanner, woraufhin der Fingerabdruck erfasst und an eine Gegenstelle übermütelt wird, wobei ein drahtloses Kommunikationsprotokoll, beispielsweise Bluetooth oder Infrarot, verwendet wird.

Das Dokument WO 2006/026794 A1 offenbart eine Vorrichtung zum Erfassen eines Fingerabdruckes, wobei die lichtempfindlichen Elemente durch eine fotoaktive Schicht auf der Basis organischer Halbleiter gebildet werden, wodurch die fotoaktive Schicht lichtdurchlässig gestaltet werden kann. Ferner ist offenbart, dass nur eine bereichsweise Beleuchtung vorgesehen sein kann, sodass nur das vom Fingerabdruck reflektierte und nicht auch das emittierte Licht erfasst wird.

Die Nachteile der aus dem Stand der Technik bekannten Verfahren liegen bspw. darin, dass sie für einen mobilen Einsatz nicht oder nur bedingt geeignet sind, da die Hand eines Benutzers bspw. in eine Vorrichtung eingelegt werden muss. Weiters von Nachteil ist es, wenn ein biometrischer Sensor in eine weitere Vorrichtung, bspw. eine Armbanduhr, integriert ist. Im täglichen Einsatz, insbesondere unter Umweltbedingungen wo mit Schmutz, Staub und erhöhter mechanischer Belastung zu rechnen ist, kann es beim bestimmungsgemäßen Einsatz der Sicherungsvorrichtung leicht zu einer Beschädigung des biometrischen Sensors kommen. Ist der biometrische Sensor nun in eine Armbanduhr integriert, muss diese derart geschützt ausgelegt sein, dass sie den Umweltbedingungen widerstehen kann und sich weiters leicht reinigen lässt. Ist der biometrische Sensors schadhaft, ist jedoch die gesamte Armbanduhr zu tauschen, was kostenintensiv und aufwendig ist, da auch die hinterlegten biometrischen Referenzwerte übertragen werden müssen bzw. neue festgelegt werden müssen.

Die Aufgabe der Erfindung liegt in einer Sicherungsvorrichtung, die eindeutige biometrische Daten auswertet und bei Erkennen eines autorisierten Benutzers, die Benützung einer Vorrichtung bzw. den Zutritt zu einer Einrichtung freigibt. Es ist weiters eine Aufgabe der Erfindung die Sicherungsvorrichtung mit einem Sensor zur Erfassung der biometrischen Daten auszustatten, der einfach und kostengünstig herzustellen ist und eine hohe Beständigkeit gegenüber Umwelteinflüssen und mechanischer Belastung aufweist. Es ist weiters Aufgabe der Erfindung eine Sicherungsvorrichtung derart modular aufzubauen, dass ein einfacher und kostengünstiger Austausch defekter bzw. fehlerhaften Komponenten möglich ist.

Die Aufgabe der Erfindung wird dadurch gelöst, dass der Sensor als elastisch nächstellbarer Dünnfilmsensor zur Erfassung biometrischer Daten bzw. spektraler Eigenschaften der Haut sowie darunter liegender Gewebeschichten ausgebildet ist, und dass die durch das Nahfeld der Haut den Benutzers gebildete Kommunikationsverbindung zu sicheren, drahtlosen Übertragung eines, von der Authentifizierungsvorrichtung ermittelten, unverwechselbaren Benutzercodes ausgebildet ist und in ihrem Wirkbereich auf einen Nahbereich, insbesondere weniger als 50cm, beschränkt ist, und dass die Verriegelungsvorrichtung bei Übereinstimmung des Benutzercodes mit einem, der Verriegelungsvorrichtung zugeordneten, Identifikationscode deaktiviert ist.

Ein Dünnfilmsensor ist im Vergleich zu bekannten biometrischen Sensoren kostengünstiger und deutlich großflächiger herstellbar, wodurch ein größerer Abschnitt eines Körperteils mit biometrischen Merkmalen erfasst werden kann. Weiters lässt sich ein Dünnfilmsensor sowohl mittels Druckverfahren als auch Aufdampfverfahren, sowie durch Kombination der beiden Verfahren herstellen. Beispielsweise haben mit einem Druckverfahren hergestellte Sensoren den Vorteil, dass die konkrete Ausbildung individuell an den geplanten Einsatz anpassbar ist. Aufdampfverfahren haben andererseits den Vorteil, dass sich nicht- oder nur schwer druckbare Materialien aufbringen lassen, wobei sich insbesondere besonders dünne Schichten ausbilden lassen.

Durch die Übertragung eines eindeutigen Benutzercodes bzw. durch die Beschränkung auf einen Nahbereich, wird ein Ausspähen des Benutzercodes zu einer späteren missbräuchlichen Verwendung weitestgehend verhindert. Der beschränkte Wirkbereich hat den weiteren Vorteil, dass es so zu keiner ungewollten Aktivierung einer benachbarten Verriegelungsvorrichtung kommen kann.

Weiters von Vorteil ist, dass die Verriegelungsvorrichtung nur bei einer festgestellten Übereinstimmung des Benutzercodes mit einem Identifikationscode deaktiviert ist. Insbesondere hat dies den Vorteil, dass sich die Verriegelungsvorrichtung bei Verlust der eindeutigen Zuordnung selbsttätig aktiviert und eine Benützung bzw. einen Zugriff auf die verriegelte Vorrichtung verhindert.

Der Sensor ist elastisch rückstellbar verformbar und passt sich somit gut an die zu erfassende Oberfläche an, ohne das es durch eine ev. Verformung zu einer Beschädigung des Sensors kommen kann. Insbesondere ist dies dann von Vorteil, wenn der Sensor an einem Körperteil angebracht wird, der durch Bewegung des Körpers kontinuierlich verformt- bzw. deformiert wird. Da sich der Sensor in diesem Fall gut an die Oberfläche anpassen kann, ist die Gefahr von fehlerhaft erfassten biometrischen Daten und damit die Gefahr einer irrtümlichen Auslösung bzw. Sperrung der Sicherungsvorrichtung wesentlich reduziert.

Die Kommunikationsverbindung ist durch das Nahfeld der Haut des Benutzers gebildet. Bei einer derartigen Kommunikationsverbindung ist eine Beeinflussung bzw. Manipulation durch Dritte weitestgehend verhindert, da die Kommunikationsverbindung den physischen Kontakt zwischen der Verriegelungsvorrichtung und der Haut des Benutzers erfordert, bspw. durch Umfassen der zu sichernden Vorrichtung. Dieser physische Kontakt lässt sich bspw. dazu benutzen, eine Entriegelung der gesicherten Vorrichtung nur solange aufrecht zu erhalten, solange die Vorrichtung vom Benutzer berührt wird. Dies hätte bspw. bei der Sicherung von Handfeuerwaffen den Vorteil, dass diese auch dann verriegelt bleibt bzw. wird, wenn sich der Benutzer im Nahbereich der Verriegelungsvorrichtung aufhält, bspw. wenn die Waffe durch einen Dritten entwendet wurde.

In einer vorteilhaften Weiterbildung kann die Kommunikationsverbindung mittels der Haut auch zur kontaktlosen Übertragung ausgebildet sein. Auf der Haut baut sich in diesem Fall ein elektrisches Feld auf, das in einem Nachbereich bspw. bis 50cm als Kommunikationsverbindung einsetzbar ist. Die Anwesenheit des Benutzers im Nahbereich der Verriegelungsvorrichtung ist somit für eine Zuordnung ausreichend.

Eine bedeutende vorteilhafte Weiterbildung erhält man, wenn der Wirkbereich der Kommunikationsverbindung einstellbar ist, da somit eindeutig festlegbar ist, in welchem Abstand zwischen der Verriegelungsvorrichtung und er Authentifizierungsvorrichtung eine eindeutige Zuordnung zwischen diesen beiden Vorrichtungen möglich ist. Insbesondere ist eindeutig festlegbar, ab welchem Abstand die Zuordnung aufgehoben wird. In einer vorteilhaften Weiterbildung kann bspw. für die Herstellung einer Zuordnung gefordert sein, dass die Authentifizierungsvorrichtung im unmittelbaren Nahbereich der Verriegelungsvorrichtung befindet. Nach hergestellter Zuordnung kann sich der Benutzer dann in einem Umkreis um die Verriegelungsvorrichtung bewegen, ohne dass die Zuordnung verloren geht.

Bspw. ist aber auch eine Annäherungserkennung möglich, wobei die Authentifizierung selbsttätig bei Annäherung an die Verriegelungsvorrichtung durchgeführt wird.

Eine besonders vorteilhafte Weiterbildung erhält man, wenn der Sensor aus zumindest einem Material der Gruppe umfassend organische halbleitende Materialien, anorganische halbleitende Materialien, Nanopartikel, gebildet ist. Diese Materialien haben den ganz besonderen Vorteil, dass sie sich in Druckverfahren bzw. Aufdampfprozessen einsetzen lassen, wodurch eine besonders effiziente und kostengünstige Herstellung des Sensors möglich ist. Insbesondere haben anspruchsgemäß ausgebildete Sensoren den Vorteil, dass sie elastisch und flexibel verformbar sind, wodurch sich der Sensor ohne die Gefahr einer Beschädigung deutlich besser an die Oberfläche des Körperteils anpassen kann. Ebenfalls von Vorteil einer anspruchsgemäßen Ausbildung ist, dass zur Herstellung deutlich weniger Energie erforderlich ist und die Entsorgungsproblematik des Sensors wesentlich geringer ist als bei konventionellen Sensoren, wodurch sich diese insbesondere als Einweg-Sensoren einsetzen lassen.

Ohne Anspruch auf Vollständigkeit seien hier als weitere verwendbare Materialien genannt: Carbon Nanotubes, PPV Poly(p-Phenyl-Vinyl), Ebenso sind auch Material-Mischformen denkbar, um bspw. die vorteilhaften Eigenschaften von organischen und anorganischen Halbleitern zu kombinieren.

Im Hinblick auf eine sichere und zuverlässige Erfassung eines benutzerspezifischen Merkmals ist es von bedeutendem Vorteil, wenn die erfassten biometrischen Daten aus der Gruppe umfassend Venenstruktur, Struktur der Hautoberfläche, Gewebestruktur, gebildet sind.

Als biometrisches Identifikationsmerkmal wird oftmals der Fingerabdruck herangezogen. Bei einem bevorzugten Einsatz der Sicherungsvorrichtung in einer Umgebung mit schwierigen Umweltbedingungen, insbesondere wo mit Schmutz zu rechnen ist, kann es leicht vorkommen, dass die Strukturen des Fingers zu stark verschmutz sind, um eindeutig erkennbar zu sein. Daher ist es von ganz besonderem Vorteil, wenn ein großflächiges biometrisches Merkmal zur eindeutigen Identifikation herangezogen werden kann. Die Oberflächenstruktur der Haut bzw. die Venenstruktur kann großflächiger und mit geringerer Auflösung abgetastet werden, wodurch selbst bei Verschmutzung der Hautoberfläche noch eine zuverlässige Erfassung der charakteristischen Merkmale möglich ist.

Wesentlich ist, dass die unterschiedlichen spektralen Eigenschaften des Gewebes gezielt ausgenützt werden, um an einer Aufnahmeposition, eine Mehrzahl eindeutiger biometrischer Merkmale aufnehmen zu können. Insbesondere werden die Reflexions- und/oder Transmissionswerte bei unterschiedlichen Spektralanteilen ausgewertet.

Von Vorteil ist auch eine Weiterbildung bei der der Sensor zumindest eine Strahlungsquelle und zumindest einen Quantendetektor umfasst, da dadurch die Erfassung biometrischer Merkmale unabhängig vom Umgebungslicht bzw. von zusätzlichen externen Beleuchtungsmitteln ist und die spektrale Empfindlichkeit der Strahlungsquelle und des Quantendetektors optimal aufeinander abgestimmt werden können.

In vorteilhaften Weiterbildungen kann die Beleuchtung der Oberfläche derart gesteuert werden, dass gezielt unterschiedliche biometrische Merkmale erfasst werden können.

Mit einer Ausbildung, bei der die Strahlungsquelle zur Abgabe von elektromagnetischer Strahlung mit einer Wellenlänge im Bereich von 350nm bis 780nm ausgebildet ist, lässt sich die Oberflächenstruktur der Haut erfassen. Die anspruchsgemäße elektromagnetische Strahlung liegt im sichtbaren optischen Bereich und ist daher in vorteilhafter Weise besonders gut zur Erfassung von Oberflächenstrukturen der Haut geeignet.

Eine weitere vorteilhafte Weiterbildung erhält man, wenn die Strahlungsquelle zur Abgabe von elektromagnetischer Strahlung mit einer Wellenlänge im Bereich von 750nm bis 1,4µm ausgebildet ist. Eine elektromagnetische Strahlung dieser Wellenlänge durchdringt die oberen Hautschichten und ermöglicht somit eine Beleuchtung tiefer liegender Merkmale, insbesondere der Venenstruktur. Die größere Eindringtiefe hat auch den weiteren Vorteil, dass ggf. auf der Hautoberfläche vorhandene Verunreinigungen die Beleuchtung der tiefer liegenden Merkmale nicht oder nur unwesentlich behindern.

In einer vorteilhaften Weiterbildung kann die Strahlungsquelle bspw. durchstimmbar ausgebildet sein, insbesondere von 650nm bis 1,4µm, wodurch sich die Eindringtiefe der elektromagnetischen Strahlung in das Gewebe gezielt steuern lässt. Durch einen entsprechend spektral selektiven Quantendetektor ist somit mit nur einem Sensor die Erfassung mehrer biometrischer Merkmale möglich. Auch kann eine Mehrfacherfassung von Merkmalen in jeweils unterschiedlicher Tiefe durchgeführt werden.

Wenn der Quantendetektor als Anordnung einer Mehrzahl fotosensitiver Elemente ausgebildet ist, lässt sich die Erfassung biometrischer Merkmale auf unterschiedliche Arten durchführen. Bspw. kann durch Zusammenschaltung mehrerer Detektoren die Empfindlichkeit erhöht werden in einem ersten Erfassungsvorgang die Grobstruktur der Hautoberfläche bzw. der Venenstruktur erfasst werden; in einem weiteren Erfassungsvorgang können dann durch eine andersartige Zusammenschaltung der fotosensitiven Elemente, hoch aufgelöste Detailmerkmale erfasst werden.

In einer Weiterbildung kann der Quantendetektor durch spektral unterschiedlich empfindliche Elemente gebildet sein. Die einzelnen Elemente können abwechselnd auf einer Trägerlage des Sensors angeordnet sein, wodurch jede Anordnung zur Erfassung charakteristischer Merkmale in einem unterschiedlichen Wellenlängenbereich ausgebildet ist. Bei entsprechend ausgebildeter Strahlungsquelle ist somit bspw. eine gleichzeitige Erfassung der Oberflächenstruktur der Haut und die Erfassung der tiefer liegenden Venenstruktur möglich.

Einen bedeutenden Vorteil erhält man, wenn der Quantendetektor aus der Gruppe umfassend organische bzw. anorganische Fotodiode, organischer bzw. anorganischer Fototransistor, Fotowiderstand gebildet ist, da sich die Bauteile aus dieser Gruppe durch ein Druckverfahren bzw. ein Aufdampfverfahren, sowie durch Kombination dieser Verfahren schnell und kostengünstig herstellen lassen. Das Einwirken einer elektromagnetischen Strahlung im optischen Bereich, führt zu einer Änderung zumindest einer elektrischen Kenngröße des Bauteils.

Im Hinblick auf einen möglichst universellen Einsatz und einen schnellen und einfachen Service bzw. Austausch des Sensors ist es von Vorteil, wenn der Sensor als Folie, insbesondere als selbst haftende Folie, ausgebildet ist. Ein anspruchsgemäß ausgebildeter Sensor lässt sich somit einfach und ohne zusätzliche Haltevorrichtung an einer, am Körper getragenen Vorrichtung anbringen.

Im Hinblick auf einen möglichst universellen Einsatz ist es von besonderem Vorteil, wenn der Sensor in eine Vorrichtung der Gruppe umfassend Armband, Kopfbedeckung, Stirnband, Halsmikrofon, Brille, Brustgurt, integriert ist bzw. an dieser angeordnet ist. Derartige Vorrichtungen sind im täglichen Gebrauch weit verbreitet bzw. werden von Personen benötigt und/oder eingesetzt, da sie Teil eines Ausrüstungsgegenstands sind. Dadurch dass sich der Sensor derart universell anordnen bzw. integrieren lässt, erhält man den besonderen Vorteil, dass für die Anbringung des Sensors zur Erfassung biometrischer Charakteristika keine aufwendigen bzw. zusätzlichen Vorrichtungen erforderlich sind. Auch lassen sich derartige Vorrichtungen besonders gut an den menschlichen Körper anpassen, wodurch ein besonders hoher Tragekomfort und eine sehr geringe Einschränkung der Bewegungsfreiheit erreicht wird.

Wenn die Authentifizierungsvorrichtung eine Energieversorgungseinrichtung umfasst, ist in besonders vorteilhafter Weise ein energieautarker Betrieb möglich. Eine Versorgung der Authentifizierungsvorrichtung mit Energie, durch eine vom Benutzer zu tragende bzw. mitzuführende externe Energiequelle ist daher in vorteilhafter Weise nicht erforderlich, wodurch die Mobilität und die Bewegungsfreiheit des Benutzers nicht eingeschränkt wird.

Die Energieversorgungseinrichtung kann durch ein elektrochemisches Element, insbesondere eine Batterie bzw. Akkumulator gebildet sein und/oder durch einen kapazitiven Energiespeicher, was den Vorteil hat, dass derartige Energieversorgungseinrichtungen weit verbreitet und bereits vielfach im Einsatz sind und somit eine besonders kostengünstige Ausbildung der Energieversorgungseinrichtung möglich ist.

In einer vorteilhaften Weiterbildung könnte die Energieversorgungseinrichtung durch ein chemisches Element gebildet sein, dass nach einer einmaligen Aktivierung eine bestimmte, technologisch bedingte Zeit, eine elektrische Energie abgibt. Der Sensor wird somit durch eine Aktivierungsaktion in Betrieb genommen, erfasst danach entsprechend der Versorgungsdauer der Energieversorgungseinrichtung biometrische Daten und wird nach Ende der Betriebsdauer entsorgt und durch einen neuen Sensor ersetzt. Gerade im Hinblick auf eine mögliche Verschmutzung des Sensors hat die Ausbildung den Vorteil, dass stets ein optimal arbeitender Sensor verwendet wird und die biometrischen Daten zuverlässig erfasst werden. Da der erfindungsgemäße Sensor besonders energiesparend ausgebildet sein kann, ist ggf. ein kapazitiver Energiespeicher bspw. ein Doppelschichtkondensator, zur Versorgung des Sensors mit elektrischer Energie ausreichend.

Eine besonders vorteilhafte Weiterbildung erhält man, wenn die Energieversorgungseinrichtung durch eine Solarzelle, insbesondere eine organische Solarzelle, gebildet ist. Durch eine Solarzelle ist ein langfristiger, energieautarker Betrieb des Sensors möglich.

Eine organische Solarzelle hat den weiteren besonderen Vorteil, dass sie mechanisch flexibel ist und sich somit ein Sensor ausbilden lässt, der sich gut an die Oberfläche des Körperteils anpassen lässt. Auch von Vorteil ist, dass organische Solarzellen kostengünstig herstellbar sind und im Hinblick auf die Entsorgung kaum Probleme bereiten, was für einen Einweg-Sensor von bedeutendem Vorteil ist.

Insbesondere ist die Energieversorgungseinrichtung dazu ausgebildet, die elektromagnetische Strahlungsquelle während der Messung mit Energie zu versorgen. Da dieser Messvorgang bevorzugt periodisch durchgeführt wird und dann ggf. nur sehr kurze Zeit in Anspruch nimmt, muss die Energieversorgungseinrichtung lediglich dazu ausgebildet sein, die Authentifizierungsvorrichtung, insbesondere die elektromagnetische Strahlungsquelle, während der Messung mit Energie zu versorgen. In den Zeiträumen zwischen den Messungen kann die Energieversorgungseinrichtung von einer externen Quelle aufgeladen werden, wobei dies bspw. durch eine Solarzelle geschehen kann. Es ist jedoch aber auch denkbar, dass der Benutzer eine kapazitätsstarke Energiequelle mit sich führt und diese, mittels einer Nahfeldübertragung über die Haut des Benutzers, in den Messpausen die Energieversorgungseinrichtung auflädt. Insbesondere haben diese Ausbildungen den ganz entscheidenden Vorteil, dass die Authentifizierungsvorrichtung besonders kompakt aufgebaut werden kann.

Biometrische Daten können an mehreren Stellen des menschlichen Körpers erfasst werden, jedoch ist eine Ausbildung von Vorteil, bei der Sensor im Bereich der Hand bzw. des der Hand zugewandten Teils des Unterarms angeordnet ist, da in diesem Abschnitt sehr charakteristische Oberflächenstrukturen erfassbar sind bzw. eine hohe Dichte an Venen vorliegt. Somit kann selbst ein kleinflächiger Sensor eine hohe Anzahl charakteristischer Merkmale erfassen und ein zuverlässiges Identifikationsmerkmal ermittelt werden.

Die erfindungsgemäße Sicherungsvorrichtung ist jedoch nicht darauf beschränkt, dass der Sensor im anspruchsgemäßen Abschnitt angeordnet ist, da auch in Kombination mit einer vorteilhaften Weiterbildung der Sensor überall dort angeordnet werden kann, wo sich charakteristische biometrische Daten erfassen lassen. Nicht einschränkende Beispiele für mögliche Anordnungen sind: am Unter- bzw. Oberarm, im Bereich des Kopfes und auch im Bereich des Oberkörpers.

Eine Ausbildung, bei der der Sensor im Bereich des Fußgelenks angeordnet ist hat den Vorteil, dass in diesem Bereich durch Bekleidungsteile ein guter Schutz des Sensors gegen mechanische Belastung und Verschmutzung möglich ist. Ein weiterer bedeutender Vorteil ist, dass ein derart angeordneter Sensor die Bewegungsfreiheit des Trägers kaum einschränkt und sich durch Bekleidungsteile gut verbergen lässt.

Es wird dabei als für den Fachmann bekannt vorausgesetzt, dass die elastisch rückstellbaren Verformungen innerhalb der materialspezifischen Grenzwerte bleiben, also das die eingeprägte Deformation zu keiner irreversiblen Materialbeschädigung führt.

Eine besonders vorteilhafte Weiterbildung erhält man, wenn der Sensor zur Erfassung von Vitalsignalen ausgebildet ist. Neben einer eindeutigen Identifikation des Trägers ist anspruchsgemäß auch eine Bestimmung lebenswichtiger Vitalzeichen möglich. Eine Authentifizierung ist somit nur von einer lebenden Person möglich, Umgehungsversuche durch Vorlegen nachgefertigter biometrischer Merkmale werde somit zuverlässig unterbunden.

In einer Weiterbildung können die erfassten Vitalsignale auch zur Warnung des Trägers vor einem lebensbedrohlichen Zustand verwendet werden. Ebenfalls ist eine Ausbildung denkbar, bei der die erfassten Vitalsignale an eine Warte übermittelt werden, um dort überwacht bzw. analysiert zu werden.

Für einen kompakten Aufbau der Sicherungsvorrichtung ist es von Vorteil, wenn das Auswerte- und Vergleichsmodul und der Sensor integriert angeordnet sind. Eine anspruchsgemäß ausgebildete Authentifizierungsvorrichtung hat den Vorteil, dass sie besonders gut integrierbar ist und sich gegenüber Umwelteinflüssen besonders gut schützen lässt. Insbesondere sind durch die technischen Möglichkeiten bekannter Integrationsverfahren besonders kompakte und robuste Vorrichtungen bzw. Module ausbildbar. Ein weiterer Vorteil ist, dass eine anspruchsgemäß ausgebildete Authentifizierungsvorrichtung die Erfassung und Auswertung der biometrischen Daten durchführt, wobei als Ergebnis dieses Vorgangs, ein unverwechselbarer Benutzercode abgegeben wird.

In einer vorteilhaften Weiterbildung ist das Auswerte- und Vergleichsmodul aus organischen halbleitenden Bauteil gebildet, wodurch sich die Vorteile organischer Halbleiter auch auf die Authentifizierungsvorrichtung übertragen lassen, insbesondere im Hinblick auf eine Einmalverwendung.

Insbesondere ist anspruchsgemäße eine Ausbildung mitumfasst, bei der bspw. ein organischer Halbleitersensor aufgedruckt wird und anorganische Halbleiterbauteile aufgebondet werden. Jedoch sind auch andere Kombinationen von organischen und anorganischen Halbleiterbauteilen durch die anspruchsgemäße Ausbildung mitumfasst.

Zur Durchführung der Auswerte- und Vergleichsoperationen ist es von Vorteil, wenn das Auswerte und Vergleichsmodul einen Speicher umfasst, da in diesem die erfassten biometrischen Daten und Zwischenergebnisse des Auswerte- und Vergleichsverfahrens abgelegt werden können.

Eine besonders vorteilhafte Weiterbildung erhält man, wenn im Speicher biometrische Referenzdaten hinterlegt sind. Zur Durchführung der Authentifizierung müssen erfasste biometrische Daten mit, diesen Benutzer eindeutig charakterisierenden, Referenzdaten verglichen werden. Sind diese Referenzdaten unmittelbar im Speicher des Auswerte- und Vergleichsmodul hinterlegt, bringt dies einen bedeutenden Vorteil im Hinblick auf die Authentifizierungssicherheit. Jede Kommunikationsverbindung zur Übertragung erfasster biometrischer Daten oder Referenzdaten birgt die Gefahr in sich, dass die Übertragung gestört bzw. verfälscht werden kann. Die anspruchsgemäße Ausbildung hat daher den ganz entscheidenden Vorteil, dass von der Erfassung der biometrischen Daten bis zur Auswertung und Vergleich mit Referenzdaten, keine sicherheitskritische Schwachstelle vorhanden ist.

Da sich aufgrund von Körperhaltung und/oder Bewegung die Position des Sensors geringfügig ändern kann und somit auch die erfassten biometrischen Daten geringfügig unterschiedlich sein werden, sind im Speicher eine Mehrzahl von Referenzdaten hinterlegt. Durch Vergleich der erfassten biometrischen Daten mit mehreren hinterlegten Referenzdaten ist somit eine sichere und zuverlässige Bestimmung der Authentizität des Benutzers möglich und die Gefahr eines fehlerhaften Auslösens bzw. Sperrens der Sicherheitsvorrichtung in vorteilhafter Weise verringert.

Entsprechend der geforderten Sicherheitsfunktion der Sicherungsvorrichtung ist es von Vorteil, wenn das Auswerte- und Vergleichsmodul kontinuierlich und/oder zeitdiskret biometrische Daten der Haut erfasst. Zur Sicherung hochsensibler Bereiche bzw. Vorrichtungen bspw. Handfeuerwaffen oder Datenverarbeitungseinrichtungen, ist eine häufige Authentifizierung erwünscht, wogegen bspw. zur Durchführung einer Zutrittskontrolle eine Authentifizierung mit geringerer Wiederholrate ausreichend ist.

In einer Weiterbildung kann die Authentifizierung auch durch einen Aktivierungsvorgang ausgelöst werden. Bspw. kann an der Authentifizierungsvorrichtung ein Betätigungselement vorhanden sein, mit dem der Authentifizierungsvorgang ausgelöst wird. Ebenso kann auch die Verriegelungsvorrichtung mit einem Fernwirkelement ausgestattet sein, dass bei Annäherung der Authentifizierungsvorrichtung an die Verriegelungsvorrichtung den Authentifizierungsvorgang auslöst.

Zur Durchführung der komplexen Authentifizierungsberechnungen ist es von Vorteil, wenn das Auswerte- und Vergleichsmodul eine Recheneinheit umfasst.

Vorteilhafte Weiterbildungen erhält man, wenn die Recheneinheit zur Auswertung der vom Sensor umfassten biometrischen Daten ausgebildet ist bzw. wenn die Recheneinheit zum Vergleich der ausgewerteten biometrischen Daten mit im Speicher hinterlegten Referenzwerten ausgebildet ist.

Diese vorteilhaften Weiterbildungen ermöglichen einen besonders kompakten Aufbau der Authentifizierungsvorrichtung, da alle Verfahren zur Erfassung und Bewertung biometrischer Daten vom Auswerte- und Vergleichsmodul durchgeführt werden.

In einer weiteren Ausbildung kann das Auswerte- und Vergleichsmodul dazu ausgebildet sein, die im Speicher hinterlegten Referenzdaten kontinuierlich zu verbessern bzw. zu erweitern, um die Erkennungssicherheit weiter zu erhöhen. Insbesondere wird dadurch die Erkennungssicherheit bei ungenauer Positionierung des Sensors verbessert. Nach erfolgter Authentifizierung des Trägers, kann in festlegbaren Zeitintervallen das biometrische Merkmal erfasst und mit den Referenzdaten verglichen werden. Kann keine eindeutige Übereinstimmung ermittelt werden, wird das neu erfasste biometrische Merkmal als weitere Referenz im Speicher abgelegt.

Zur Erreichung einer höchstmöglichen Sicherheit der Benutzerauthentifikation ist es von Vorteil, wenn die Authentifizierungseinrichtung zur kontinuierlichen und/oder zeitdiskreten Abgabe eines unverwechselbaren Benutzercodes ausgebildet ist. Da dieser Benutzercode von der Authentifizierungsvorrichtung an die Verriegelungsvorrichtung übertragen werden muss, wodurch eine sicherheitstechnische Schwachstelle entsteht, ist es von ganz entscheidender Bedeutung, wenn der Benutzercode derart ausgebildet ist, dass eine Verfälschung bzw. Manipulation zuverlässig verhindert wird. Bspw. kann der Benutzercode durch ein Verschlüsselungsverfahren nach einem Einwegcode ausgebildet sein. Wie bereits erwähnt, können unterschiedliche Sicherheitsanforderungen eine kontinuierliche bzw. zeitdiskrete Authentifizierung verlangen.

Da die Kommunikationsverbindung einen Angriffspunkt für Manipulation bzw. Verfälschung darstellt, ist es von Vorteil, wenn die darüber übertragenen Daten derart gesichert sind, dass eine Verfälschung bzw. Manipulation der Daten weitestgehend verhindert wird und/oder ein derart missbräuchlicher Vorgang von der Verriegelungsvorrichtung zuverlässig erkannt wird. Insbesondere bedeutet dies, dass eine in missbräuchlicher Absicht aufgezeichnete Übertragung bei einer erneuten Übertragung als missbräuchliche Verwendung erkannt bzw. abgewiesen wird.

Im Hinblick auf eine möglichst lange Einsatzdauer der Authentifizierungsvorrichtung ist es von Vorteil, wenn diese einen Energiesparzustand aufweist, in dem der Energieverbrauch kleiner als 500µW ist. Gerade bei autark mit Energie versorgten Vorrichtungen ist es von entscheidender Bedeutung, wenn die Vorrichtung nur im aktiven Zustand, also bei Durchführung einer Authentifikation, Energie verbraucht und die restliche Zeit möglichst wenig Energie verbraucht. '

Insbesondere von Vorteil ist, wenn die Authentifizierungsvorrichtung nur periodisch zur Durchführung der Messung in einen aktiven Betriebszustand versetzt wird und die restliche Betriebszeit in einem Energiesparzustand verharrt, wodurch sich eine bedeutende Verlängerung der Betriebszeit erreichen lässt.

Ein derart geringer Energieverbrauch ist besonders im Hinblick auf die zulässige Belastung des menschlichen Körpers mit elektromagnetischen Feldern von Vorteil. Da gemäß einer vorteilhaften Weiterbildung der Energiespeicher der Authentifizierungseinrichtung in den Messpausen wieder geladen wird, ist es von ganz besonderem Vorteil, wenn der Eigenverbrauch der Authentifizierungseinrichtung anspruchsgemäß gering ist, da dann über die durch die Haut gebildete Kommunikationsverbindung ausreichend Energie in den Energiespeicher der Authentifizierungseinrichtung übertragen werden kann, ohne das eine Gefährdung der Person besteht. Die International Radiation Protection Association (IRPA) hat diesbezüglich Grenzwerte für Feldstärken festgelegt, die auf den menschlichen Körper einwirken dürfen. Insbesondere wurde für die Spezifische Absorptionsrate (SAR) ein Grenzwert von 80mW/kg und für die Stromdichte (S) ein Grenzwert von 250mA/m² (rms) festgelegt.

Durch den anspruchsgemäß geringen eigenen Energieverbrauch der Authentifizierungseinrichtung ist eine für den menschlichen Organismus sichere und dennoch ausreichende Versorgung der Energieversorgungseinrichtung der Authentifizierungseinrichtung mit elektrischer Energie gegeben.

Für eine zuverlässige Authentifikation ist es von Vorteil, wenn die Authentifizierungsvorrichtung ein drahtloses Positionsortungssystem aufweist. Beispielsweise kann dies durch GPS oder dGPS gebildet sein, es ist aber auch jedes andere drahtlose Positionsortungssystem einsetzbar. In einem Abschnitt mit mehreren Vorrichtungen die eine Authentifikation erfordern, kann die Authentifizierungsvorrichtung durch Kenntnis der aktuellen Aufenthaltsposition gezielt eine Authentifikation mit einer speziellen Vorrichtung auslösen. Beispielsweise ist es anspruchsgemäß möglich, dass sich der Träger, nach einmaliger Authentifikation, in einem festgelegten Abschnitt bewegen kann, ohne die Zuordnung zu verlieren.

In einer Weiterbildung ist es aber auch denkbar, die Position der Authentifizierungsvorrichtung kontinuierlich zu überwachen, um bspw. den Bewegungsverlauf zu protokollieren, oder bei Verlassen und/oder Annäherung an einen Bereich, eine Warnmeldung auszulösen.

Neben der Kommunikationsverbindung zwischen der Authentifizierungsvorrichtung und der Verriegelungsvorrichtung kann die Authentifizierungsvorrichtung auch ein Netzwerk-Kommunikationsmodul aufweisen. Dieses Netzwerk-Kommunikationsmodul kann zur Herstellung einer Kommunikationsverbindung mit einer Datengegenstelle ausgebildet sein. Die erfassten biometrischen Daten können somit über diese, bevorzugt drahtlose, Kommunikationsverbindung an eine Verwaltungs- oder Überwachungseinrichtung übermittelt werden.

Gemäß einer vorteilhaften Weiterbildung können auch erfasste Vitalsignale über diese Kommunikationsverbindung an eine Verwaltungs- oder Überwachungseinrichtung übermittelt werden.

Eine ganz besonders vorteilhafte Weiterbildung erhält man, wenn die Authentifizierungsvorrichtung ein Verschlusselement aufweist, das zur Abgabe eines Triggersignals ausgebildet ist. Beim Anbringen der Authentifizierungsvorrichtung am Körperteil wird diese durch Aktivierung bzw. Verriegelung des Verschlusselements kraft- bzw. formschlüssig fixiert. Die Aktivierung bzw. Verriegelung bewirkt auch eine Triggerung des Authentifizierungsvorgangs.

Im Hinblick auf eine Energieeinsparung ist es von entscheidendem Vorteil, wenn die Erfassung biometrische Merkmale nicht kontinuierlich durchgeführt wird. Durch die Triggerung ist festgestellt, dass der Träger die Authentifizierungsvorrichtung, insbesondere den Sensor, über dem zu erfassenden biometrischen Merkmal positioniert hat. Danach wird das biometrische Merkmal eindeutig erfasst und bei eindeutiger Erkennung die Zuordnung mit der Verriegelungsvorrichtung initiiert. Solange das Verschlusselement aktiviert bzw. verriegelt ist, braucht das biometrische Merkmal nicht laufend erfasst werden, es reicht dann bspw. eine periodische Erfassung. Es ist aber auch möglich, dass die Authentifizierung bis zur Abnahme der Authentifizierungseinrichtung erhalten bleibt, also keine weitere Erfassung erforderlich ist.

Ein zusätzlicher Schutz gegen eine missbräuchliche Verwendung erreicht man, wenn die Authentifizierungsvorrichtung ein Authentizitätsmerkmal aufweist. Beispielsweise könnten die vom Sensor erfassten Daten aufgezeichnet werden und dem Auswerte- und Vergleichsmodul zur Auswertung präsentiert werden, um eine missbräuchliche Zuordnung zu einer Verriegelungsvorrichtung zu erreichen. Das Authentizitätsmerkmal ist bevorzugt derart ausgebildet, dass jede Manipulation an der Authentifizierungsvorrichtung eindeutig erkennbar ist, bspw. das es bei einer Manipulation nicht wieder herstellbar zerstört wird.

In einer vorteilhaften Weiterbildung kann das Authentizitätsmerkmal auch derart ausgebildet sein, dass eine Manipulation an der Authentifizierungsvorrichtung eine Zerstörung des Auswerte- und Vergleichsmodul bewirkt, insbesondere der, gemäß einer vorteilhaften Weiterbildung, im Speicher hinterlegten Referenzdaten.

Ein weites Merkmal zur eindeutigen Authentifikation eines Trägers erhält man, wenn die Authentifizierungsvorrichtung eine Längenmessvorrichtung aufweist. Die Authentifizierungsvorrichtung wird über einem Körperabschnitt kraft- bzw. formschlüssig angeordnet, wobei ggf. ein Umfang zu umfassen ist. Die Messung der zu umfassenden Länge, kann als weiteres Merkmal zur eindeutigen Authentifikation des Trägers herangezogen werden. Die Längenmessvorrichtung kann durch jede Vorrichtung gebildet sein, die zur Bestimmung des Abstands zweier Punkte bzw. zur Veränderung des Abstands geeignet ist und sich an bzw. in einer Authentifizierungsvorrichtung anordnen lässt.

Beispielsweise kann die Längenmessvorrichtung als resitiv wirkendes Messband ausgebildet sein, bei dem sich eine Änderung der Länge in einer Änderung des Gesamtwiderstands auswirkt.

Im Hinblick auf die Sicherung des übertragenen Benutzercodes ist es weiters von Vorteil, wenn die Authentifizierungs- und Verriegelungsvorrichtung eine Ver- und/oder Entschlüsselungseinheit aufweisen. Bei anspruchsgemäßer Ausbildung werden die übertragenen Daten, zusätzlich zu ggf. anderen kommunikationstechnischen Sicherungsverfahren, derart geschützt, bspw. durch Einwegcodes oder auch Public-Key-Systeme, dass selbst bei einer missbräuchlichen Aufzeichnung der Kommunikation, nicht oder nur sehr schwer auf den Inhalt der Kommunikation rückgeschlossen werden kann.

Der übertragene Benutzercode wird in der Verriegelungsvorrichtung geprüft und bei Übereinstimmung mit einem Identifikationscode wird anspruchsgemäß eine zeitbegrenzte Zuordnung der Verriegelungsvorrichtung zur Authentifizierungsvorrichtung hergestellt. Die anspruchsgemäße Ausbildung hat den Vorteil, dass eine einmal hergestellte Zuordnung selbstständig wieder gelöscht wird, wodurch bei erneutem Zugriff auf die gesicherte Vorrichtung eine erneute Authentifizierung erforderlich ist. Diese Ausbildung stellt somit in vorteilhafter Weise sicher, dass eine entriegelte Vorrichtung nicht unbeabsichtigt und unbeaufsichtigt entriegelt bleibt und somit der Manipulation durch Dritte ausgesetzt ist. Stellt ein Benutzer eine Zuordnung zu einer gesicherten Vorrichtung her und bewegt sich dann von dieser weg, insbesondere aus dem Wirkbereich der Kommunikationsverbindung, würde sich diese nach einer einstellbaren Zeitspanne selbstständig verriegeln und somit eine Manipulation durch Dritte verhindern.

In einer vorteilhaften Weiterbildung kann die Verriegelungsvorrichtung dazu ausgebildet sein, den Benutzer vor Verlust der Zuordnung darauf hinzuweisen, wodurch dieser bspw. eine erneute Authentifikation auslösen kann. In einer anderen Weiterbildung kann die Verriegelungsvorrichtung dazu ausgebildet sein, mittels eines Fernwirkmittels bzw. über die Kommunikationsverbindung von der Authentifizierungsvorrichtung eine erneute Authentifizierung anzufordern.

Bei einer hergestellten Zuordnung kann der Benutzer an der freigegebenen Vorrichtung Einstellungen vornehmen, sowie diese an seine Bedürfnisse anpassen. Um diese Änderungen bzw. Anpassungen nicht irrtümlich zu verlieren ist es von Vorteil, wenn bei einer aktiven Zuordnung, die Zuordnung einer weiteren Authentifzierungsvorrichtung nicht möglich ist. Eine authentifiziert freigegebene Vorrichtung kann somit nur von einem Benutzer gleichzeitig verwendet werden. Der zugeordnete Benutzer muss daher aktiv die Zuordnung aufheben, um die Vorrichtung für einen weiteren Benutzer freizugeben.

Eine Verriegelungsvorrichtung die eine Sicherungselement und ein Antriebsmittel umfasst hat den Vorteil, dass sich bei nicht hergestellter Zuordnung eine eindeutige Ruhestellung des Sicherungselements ausbilden lässt. Bevorzugt ist die Verriegelungsvorrichtung derart ausgebildet, dass im Ruhezustand die Verriegelungsvorrichtung inaktiv ist, also die Vorrichtung verriegelt ist und somit ein Zutritt oder Zugriff nicht gestattet ist bzw. dass eine Verwendung der Vorrichtung nicht möglich ist. Dies ist von bedeutendem Vorteil, da somit eine missbräuchliche Verwendung bzw. Benutzung durch gezielte Manipulation der Verriegelungsvorrichtung unterbunden wird. Beispielsweise ist es somit nicht möglich, durch Unterbrechen der Energieversorgung einer freigegeben Verriegelungsvorrichtung, missbräuchlich einen dauerhaft ungesicherten Zustand herzustellen.

Das Sicherungselement ist bevorzugt durch einen Sicherungsbolzen gebildet, jedoch können alle Sicherungselemente eingesetzt werden, die eine mechanische Verriegelung bzw. Fixierung gestatten. Das Antriebsmittel kann bspw. durch einen Stellantrieb gebildet sein, jedoch sind auch hier alle Ausführungen denkbar, die zur Bewegung eines Sicherungselements geeignet sind.

In einer Weiterbildung kann die Verriegelungsvorrichtung aber auch durch elektronische bzw. datentechnische Verriegelungselemente gebildet sein, bspw. durch Anmeldemasken, die einen Zugriff erst nach erfolgter Authentifikation ermöglichen.

In einer vorteilhaften Weiterbildung ist das Antriebsmittel der Verriegelungsvorrichtung zur Bewegung des Sicherungselements zwischen einer verriegelten und einer entriegelten Position ausgebildet. Ein bewegbares Sicherungselement bietet den entscheidenden Vorteil, dass sich dieses besonders gut gegen Manipulation und missbräuchliche Betätigung schützen lässt. Insbesondere kann dieses in einer zu sichernden Vorrichtung derart angeordnet sein, dass es von einem Außenraum nicht erreichbar ist. Bei erfolgreich durchgeführter Authentifikation wird das Sicherungselement von der verriegelten in die entriegelte Position bewegt und somit die gesicherte Vorrichtung freigegeben bzw. der Zutritt gestattet.

Im Zuge der Weiterentwicklung von Feuerwaffen, insbesondere von Faustfeuerwaffen, wird auch der mechanische Zündvorgang immer mehr abgelöst. Gemäß einer Weiterbildung ist daher die Verriegelungsvorrichtung durch einen elektronischen Zündgeber gebildet was den ganz besonderen Vorteil hat, das sich außerordentlich hohe Zündwiederholraten realisieren lassen, da keine oder kaum bewegte Teile für den Zündvorgang erforderlich sind. Diese hohe Zündgeschwindigkeit bei gleichzeitiger deutlicher Steigerung der Verwendungssicherheit durch befugte Personen, sind ganz besondere Vorteile der anspruchsgemäßen Ausbildung. Wesentlich von Vorteil ist, dass die Verriegelungsvorrichtung deutlich kompakter ausgeführt sein kann, da eine Vielzahl bislang erforderlicher Komponenten wegfallen, was auch einen besonderen Vorteil im Hinblick auf die Zuverlässigkeit darstellt.

In einer vorteilhaften Weiterbildung könnte die Verriegelungsvorrichtung auch dahingehend ausgebildet sein, dass der Versuch einer missbräuchlichen Verwendung durch Unbefugte einen Schutzmechanismus auslöst. Dieser könnte bspw. die Verriegelungsvorrichtung unbrauchbar machen, oder auch eine schmerzhafte Rückmeldung an den Unbefugten auslösen, bspw. einen Elektroschock.

Einen bedeutenden Sicherheitsgewinn erreicht man, wenn die Verriegelungsvorrichtung eine Statusanzeige aufweist. Für den Benutzer einer erfindungsgemäßen Sicherungsvorrichtung ist es von Vorteil, wenn der Verriegelungsstatus der Verriegelungsvorrichtung klar, schnell und eindeutig erkennbar ist. Am Beispiel einer Faustfeuerwaffe könnte die anspruchsgemäße Ausbildung bspw. durch eine Leuchtvorrichtung am Laufende gebildet sein, die einen stark gerichteten Lichtstrahl abgibt. Dieser Lichtstrahl kann bspw. als Zielhilfe genutzt werden und ist in diesem Fall dann aktiv, wenn die Verriegelungsvorrichtung deaktiviert ist. Bei einer missbräuchlichen Verwendung könnte eine Waffe bspw. auf den autorisierten Benutzer gerichtet werden, worauf dieser aufgrund der anspruchsgemäßen Ausbildung sofort erkennen kann, ob die Verriegelungsvorrichtung aktiv oder deaktiv ist und somit die entsprechenden Verteidigungsschritte setzten.

Die Erfindung wird im Nachfolgenden anhand der in den Zeichnungen dargestellten Ausführungsbeispiele näher erläutert.

Es zeigen jeweils in schematisch vereinfachter Darstellung:
- Fig. 1: ein Blockschaltbild der erfindungsgemäßen Sicherungsvorrichtung;
- Fig. 2: mögliche Positionen am menschlichen Körper zur Anordnung des Sensors zur Erfassung biometrischer Daten;
- Fig. 3: a) und b) eine Vorder- und Rückseite einer möglichen Ausbildung der Authentifizierungsvorrichtung;
- Fig. 4: a) und b) eine Vorder- und Rückseite einer weiteren möglichen Ausbildung der Authentifizierungsvorrichtung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese bei einer Lageänderung sinngemäß auf die neue Lage zu übertragen. Weiters können auch Einzelmerkmale oder Merkmalskombinationen aus den gezeigten und beschriebenen, unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

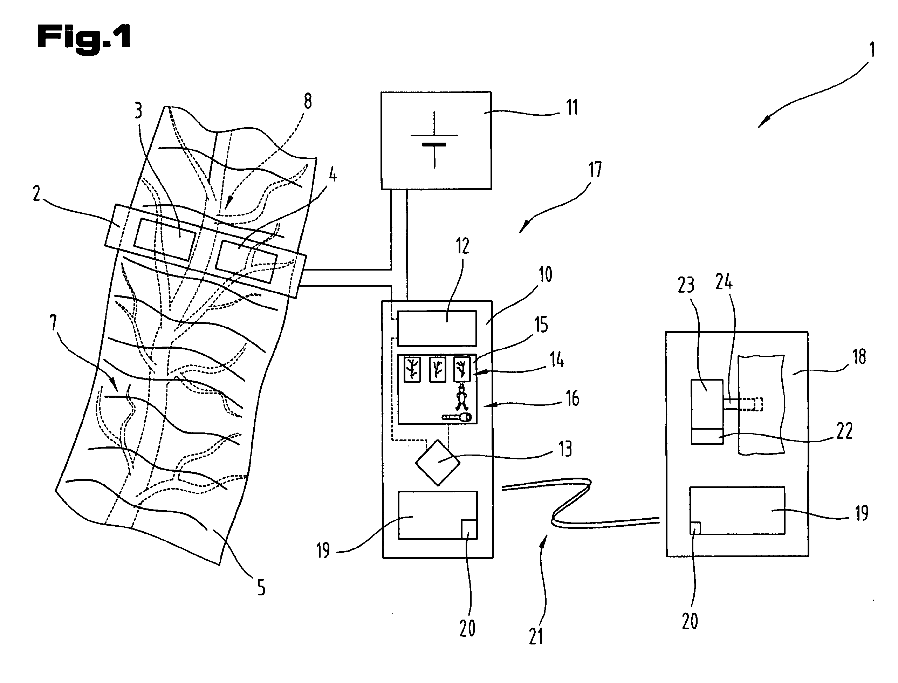

Fig. 1 zeigt ein Bockschaltbild der erfindungsgemäßen Sicherungsvorrichtung 1. Ein Sensor 2, umfassend eine Quelle 3 für elektromagnetische Strahlung und einen Quantendetektor 4, ist über einem Körperteil 5 angeordnet. Bevorzugt wird der Sensor 2 über jenen Abschnitten von Körperteilen angeordnet, in denen eine Mehrzahl charakteristischer biometrischer Daten vorhanden sind. Bspw. im Bereich des Unterarms 6 lässt sich eine charakteristische Struktur der Hautoberfläche 7 und/oder eine charakteristische Venenstruktur 8 erfassen.

Der Sensor 2 und das Auswerte- und Vergleichsmodul 10 werden von einer Energieversorgungseinrichtung 11 mit elektrischer Energie versorgt, wobei ggf. auch zwei eigenständige Energieversorgungseinrichtungen, zur getrennten Versorgung des Sensors 2 und der Auswerte- und Vergleichsmodul 10, vorhanden sein können. Die vom Sensor ermittelten biometrischen Daten werden an das Auswerte- und Vergleichsmodul 10 übermittelt und von der Recheneinheit 12 ausgewertet. Die ausgewerteten biometrischen Daten werden von einem Vergleichsmodul 13 mit biometrischen Referenzdaten 14, die im Speicher 15 hinterlegt sind, verglichen. Bei Übereinstimmung wird aus hinterlegten personenspezifischen Daten 16 ein eindeutiger, unverwechselbarer Benutzercode erzeugt.

Die Authentifizierungsvorrichtung 17 und die Verriegelungsvorrichtung 18 weisen jeweils ein Kommunikationsmodul 19 auf, das ggf. zusätzlich ein Ver- und/oder Entschlüsselungsmodul 20 aufweisen kann. Das Kommunikationsmodul 19 ist zur Herstellung einer drahtlosen Nahbereichs-Kommunikationsverbindung 21 zwischen der Authentifizierungsvorrichtung 17, insbesondere dem Auswerte- und Vergleichsmodul 10, und der Verriegelungsvorrichtung 18 ausgebildet. Die Verriegelungsvorrichtung 18 weist weiters ein Auswertemodul 22 auf, das den, über die Kommunikationsverbindung 21 übertragenen, eindeutigen und unverwechselbaren Benutzercode mit einem, der Verriegelungsvorrichtung zugeordneten Identifikationscode vergleicht und bei Übereinstimmung ein Antriebsmittel 23 derart ansteuert, dass dieses ein Sicherungselement 24 von einer verriegelten in eine entriegelte Position bewegt.

Bei der Erfassung biometrischer Daten, insbesondere bei kontinuierlicher bzw. periodischer Erfassung, muss sich der Sensor 2 möglichst gut an die Oberflächenform des Körperteils anpassen können, um die biometrischen Daten der Hautoberflächenstruktur 7 bzw. der Venenstruktur 8 eindeutig erfassen zu können. Der wesentliche Vorteil eines erfindungsgemäß ausgebildeten Dünnfilmsensors liegt nun darin, dass dieser flexibel und elastisch rückstellbar deformierbar ist, wodurch eine besonders gute Anpassung an die Oberflächenform des zu erfassenden Abschnitts eines Körperteils möglich ist. In einer vorteilhaften Weiterbildung ist der Sensor 2, insbesondere die Quelle 3 und der Quantendetektor 4 aus einem organischen halbleitenden Material gebildet, wodurch sich der Sensor besonders kostengünstig herstellen lässt, insbesondere sind so genannte Einweg-Sensoren ausbildbar. Besonders in Hinblick auf einen Einsatz des Sensor in schwierigen Umweltbedingungen bspw. wenn mit einem starken Schmutzaufkommen zu rechnen ist, ist es von besonderem Vorteil, wenn der Sensor zur Erfassung biometrischer Daten nach einmaligem Gebrauch ohne Probleme für die Umwelt entsorgt werden kann und durch einen neuen, kostengünstigen Sensor ersetzt werden kann.

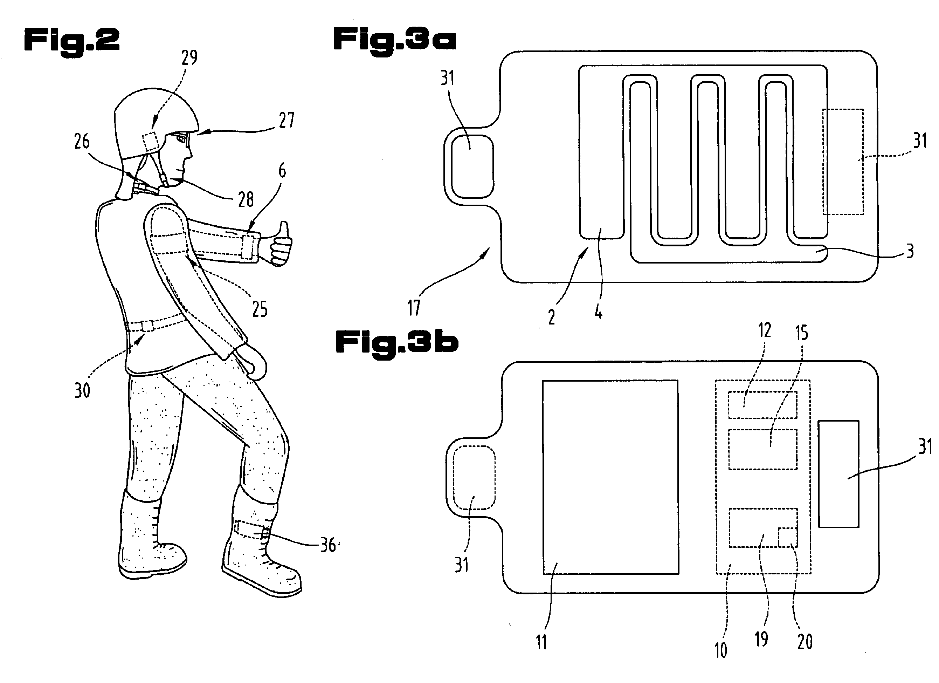

Je nach zu erfassenden biometrischen Merkmal wird von der Quelle 3 eine elektromagnetische Strahlung mit einer bestimmten Wellenlänge abgegeben. Zur Erfassung von Hautoberflächenstrukturen 7 ist eine Beleuchtung der Haut im optischen sichtbaren Bereich, insbesondere im Wellenlängenbereich von 650nm bis 780nm ausreichend. Soll jedoch die tiefer in der Haut liegende Venenstruktur 8 erfasst werden, muss zur Beleuchtung eine elektromagnetische Strahlung mit einer längeren Wellenlänge verwendet werden, insbesondere im Bereich von 750nm bis 1,4µm, da diese in der Lage ist, weiter in die Haut einzudringen und somit die tiefer liegenden Venenstrukturen zu beleuchten. Der Quantendetektor 4 ist bevorzugt durch eine Mehrzahl fotosensitiver Elemente gebildet, die bspw. schachbrettartig angeordnet sind, wobei die Größe der einzelnen fotosensitiven Elemente, das maximal erzielbare Auflösungsvermögen bestimmt. Gegebenenfalls kann auch die Quelle 3 durch eine Mehrzahl einzelner, elektromagnetische Strahlung abgebende Elemente gebildet sein, die abwechselnd mit Detektorelementen integriert angeordnet werden können. Der Vorteil dieser Anordnung liegt darin, dass eine gleichmäßige Beleuchtung des zu erfassenden Abschnitts durch die verteilt angeordneten Leuchtelemente erreicht wird. Weitere Anordnungsmöglichkeiten von Quelle und Detektor werden in Fig. 3 beschrieben.

Zur Versorgung des Sensors 2 mit elektrischer Energie, ist die Energiequelle 11 bevorzugt am Sensor 2 angeordnet und durch ein chemisches Element gebildet. In einer vorteilhaften Weiterbildung könnte diese Energiequelle 11 aber auch als Solarzelle ausgebildet sein, insbesondere als organische Solarzelle. In einer anderen Weiterbildung könnte das chemische Element bspw. zum Einmalgebrauch ausgebildet sein. Dabei wird das chemische Element durch ein Aktivierungsmittel aktiviert und stellt hernach für eine, technologisch bedingte Zeit, elektrische Energie zur Verfügung. Wiederum im Hinblick auf eine Einmalverwendung des Sensors ist eine derartige Ausbildung von besonderem Vorteil.

In einer Weiterbildung kann die Energiequelle 11 auch gemeinsam mit dem Auswerte- und Vergleichsmodul 10 am Sensor 2 angeordnet sein, wodurch sich in vorteilhafter Weise eine sehr kompakte und hoch integrierte Authentifizierungsvorrichtung 17 ausbilden lässt.

Im Speicher 15 des Auswerte- und Vergleichsmodul 10 sind bevorzugt mehrere biometrische Referenzdaten 14 hinterlegt. Da sich aufgrund unterschiedlicher Positionierung des Sensors und variierendem Auflagedruck, die erfassten biometrischen Daten geringfügig ändern können, ist es von Vorteil, wenn das Vergleichsmodul 13 eine Mehrzahl biometrischer Referenzdaten 14 als Vergleichsmöglichkeit zur Verfügung hat. Diese Referenzdaten werden bspw. in einem Einlernvorgang ermittelt, wo der Sensor 2 im Erfassungsabschnitt positioniert wird und für jeweils eine geringfügig geänderte Position bzw. einen geringfügig geänderten Anpressdruck ein Referenzmuster aufgenommen wird.

Zusätzlich kann im Auswerte- und Vergleichsmodul 10, insbesondere im Speicher 15, ein personenspezifischer Datensatz 16 hinterlegt sein. Bei Übereinstimmung des erfassten biometrischen Merkmals mit den hinterlegten Referenzdaten wird aus dem personenspezifischen Datensatz eine eindeutige, unverwechselbare Benutzerkennung, insbesondere ein Benutzercode, generiert. Dieser Benutzercode ist derart ausgebildet, dass einerseits eine eindeutige Authentifizierung möglich ist und dass andererseits eine Fälschung bzw. Manipulation des Codes verhindert wird. Da zwischen der Authentifizierungsvorrichtung 17 und der Verriegelungsvorrichtung 18 eine bevorzugt drahtlose Kommunikationsverbindung 21 besteht, könnte der übertragene Benutzercode von Dritten aufgezeichnet werden und hernach in missbräuchlicher Absicht zur Herstellung einer Zuordnung zur Verriegelungsvorrichtung 18 eingesetzt werden. Durch die speziellen Eigenschaften des Codes ist jedoch sichergestellt, dass eine missbräuchliche Zuordnung durch Dritte zuverlässig verhindert wird.

Die Kommunikationsverbindung 21 ist durch eine im Nahbereich wirkende Kommunikationsverbindung gebildet . Da drahtlose Kommunikationsverbindungen Dritten zugänglich sind und somit eine Verfälschung aber auch eine störende Beeinflussung durch Dritte möglich ist, können ggf. die übertragenen Daten zusätzlich durch ein Ver- und/oder Entschlüsselungsmodul derart verschlüsselt werden, dass selbst bei einer missbräuchlichen Aufzeichnung und Analyse der übertragenen Daten, nicht auf den Inhalt der übertragenen Information zurück geschlossen werden kann. Diese Verschlüsselung kann durch Einwegcodes, aber auch durch so genannte Public-Key-Systeme gebildet sein.

Die Kommunikationsverbindung 21 ist als eine Nahfeldkommunikation über die Haut des Benutzers ausgebildet. Eine derartige Ausbildung hat den Vorteil, dass die Person sich in einem bestimmten Umkreis um die Verriegelungsvorrichtung aufhalten muss bzw. diese berühren muss. Außerdem ist hie ein besonders guter Schutz gegenüber einer Beeinflussung der Übertragung durch Dritte gegeben, da sich durch geeignete Wahl der Übertragungsparameter, die Reichweite der Übertragung gut begrenzen lässt.

Die Verriegelungsvorrichtung 18 ist derart ausgebildet, dass in einer Ruhestellung, also ohne aktive Zuordnung eines authentifizierten Benutzers bzw. bei Verlust der Zuordnung, sich das Sicherungselement 24 in einer verriegelten Position befindet. Das Auswertemodul 22 hat die Aufgabe, den übertragenen Benutzercode zu analysieren und bei einer entsprechend Übereinstimmung mit einem Identifikationscode, das Antriebsmittel 23 derart anzusteuern, dass das Sicherungselement 24 in eine entriegelte Position gebracht wird. Die Berechtigungsfreigaben bzw. Identifikationscodes können bspw. im Auswertemodul 22 hinterlegt sein und legen fest, welche Benutzercodes eine Zuordnung mit der Verriegelungsvorrichtung herstellen dürfen.

Fig. 2 zeigt mögliche Positionen für die Anordnung des Sensors bzw. der Authentifizierungsvorrichtung. Eine Anordnung am Unterarm 6 hat den Vorteil, dass in diesem Abschnitt eine hohe Venendichte mit unterschiedlicher Struktur sowie eine Mehrzahl unterschiedlicher Hautoberflächenstrukturen erfassbar sind. An dieser Position besteht jedoch die Gefahr von erhöhter mechanischer Belastung, weiters ist mit dem Einwirken von Schmutz bzw. Staub zu rechnen.

Eine Anordnung am Oberarm 25 ist aufgrund einer zu erwartenden geringeren mechanischen Belastung bzw. einer geringeren Verschmutzungsgefahr von Vorteil.

Ebenso sind Anordnungen von Vorteil, bei denen Sensor und/oder die Authentifizierungsvorrichtung im Bereich des Kopfes einer Person angeordnet ist. Beispielsweise ist eine Anordnung im bzw. an einem Halsmikrofon 26 oder dem Bügel eines Augenschutzes 27 möglich. Trägt der Benutzer ein Kopfbedeckung, bspw. einen Helm oder eine Kappe, so besteht die Möglichkeit der Anordnung am Halteriemen 28 oder an der Innenseite der Kopfbedeckung 29. Weiters besteht die Möglichkeit, dass der Sensor bzw. die Authentifizierungsvorrichtung an einem Gurt 30 angeordnet ist, wobei dieser Gurt bspw. auch als Brustgurt getragen wird.

Diese Aufzählung der möglichen Anordnungspositionen ist nicht einschränkend zu sehen, insbesondere kann der Sensor bzw. die Authentifizierungsvorrichtung an all jenen Abschnitten des menschlichen Körpers angeordnet sein, in denen sich charakteristische biometrische Daten erfassen lassen.

Fig. 3a und 3b zeigen eine beispielhafte Ausbildung einer Authentifizierungsvorrichtung 17. Fig. 3a zeigt die Ansicht auf den Sensor 2, wobei diese Seite üblicherweise auf der Hautoberfläche aufliegt.

Die Authentifizierungsvorrichtung 17 ist in der dargestellten Ausbildung als eine Art Manschette ausgebildet, die flexibel um einen Körperteil, bspw. einen Arm, angelegt werden kann. Durch das Verschlusselement 31 wird die Manschette um das Körperteil fixiert. Fig. 3a zeigt weiters eine beispielhafte Anordnung des Sensors 2, insbesondere der Quelle 3 für elektromagnetische Strahlung und des Quantendetektors 4. Diese sind fingerförmig ineinander verschränkt angeordnet, wobei jeweils ein Finger der Strahlungsquelle 3 den benachbarten, vom Detektor 4 erfassten Abschnitt beleuchtet. Neben dieser beispielhaften Anordnung sind alle anderen Anordnungen denkbar, bei denen ein Abschnitt der Hautoberfläche eines Körperteils von einer Strahlungsquelle beleuchtet wird und das Bild des beleuchteten Abschnitts von einem Quantendetektor aufgenommen wird. Insbesondere sind Anordnungen von Vorteil, bei denen der erfasste Abschnitt möglichst gleichmäßig ausgeleuchtet wird, wobei es durch die Anordnung der Strahlungsquelle zu einer möglichst geringen Beeinflussung bzw. Einschränkung des Detektorbereichs kommt.

Auf der zweiten Oberfläche ist wie in Fig. 3b dargestellt, bspw. die Energieversorgungseinrichtung 11 angeordnet. Gegebenenfalls kann auch noch das Auswerte- und Vergleichsmodul 10 angeordnet sein, in einer vorteilhaften Weiterbildung kann dieses jedoch auch in der Manschette geschützt angeordnet sein. Die Energieversorgungseinrichtung 11 kann gemäß einer besonders vorteilhaften Weiterbildung durch eine organische Solarzelle gebildet sein. Diese Ausbildung hat den Vorteil, dass ein energieautarker Betrieb der Authentifizierungsvorrichtung 17 möglich ist. Organische Solarzellen haben den bedeutenden Vorteil, dass sie flexibel sind und sich somit beim Anbringen der Manschette sehr gut an die Oberflächenform des Körperteils anpassen können. Organische Solarzellen, insbesondere haben alle Bauteile aus organischen halbleitenden Material den weiteren Vorteil, dass ihre Entsorgung bedeutend weniger Probleme bereitet als die Entsorgung von Vorrichtungen bzw. Bauteilen aus anorganischem halbleitendem Material.

In einer Weiterbildung können der Sensor und das Auswerte- und Vergleichsmodul, insbesondere einzelne Elemente davon, auch getrennt voneinander angeordnet sein. Bspw. ist eine Anordnung des Auswerte- und Vergleichsmoduls am bzw. in einem Ausrüstungsgegenstand denkbar, die erfassten biometrischen Daten werden über ein Kommunikationsmittel, bevorzugt ein drahtloses, vom Sensor zum Auswerte- und Vergleichsmodul übertragen. Bei Beschädigung bzw. Verschmutzung des Sensors wird dieser einfach und unkompliziert gegen einen neuen ausgetauscht. Auch ist eine Ausbildung denkbar, bei dem der Speicher, in dem die biometrischen Referenzdaten hinterlegt sind, koppelbar mit dem Auswerte- und Vergleichsmodul verbunden ist. Diese Ausbildung hat ebenfalls den Vorteil, dass bei einer Fehlfunktion der Authentifizierungsvorrichtung, der Speicher mit den hinterlegten Referenzdaten abgekoppelt und in eine neue Authentifizierungsvorrichtung eingesetzt wird. Dadurch ist ein schneller und unkomplizierter Wechsel einer defekten Authentifizierungsvorrichtung möglich, ohne dass biometrische Referenzdaten erneut eingelernt werden müssten.

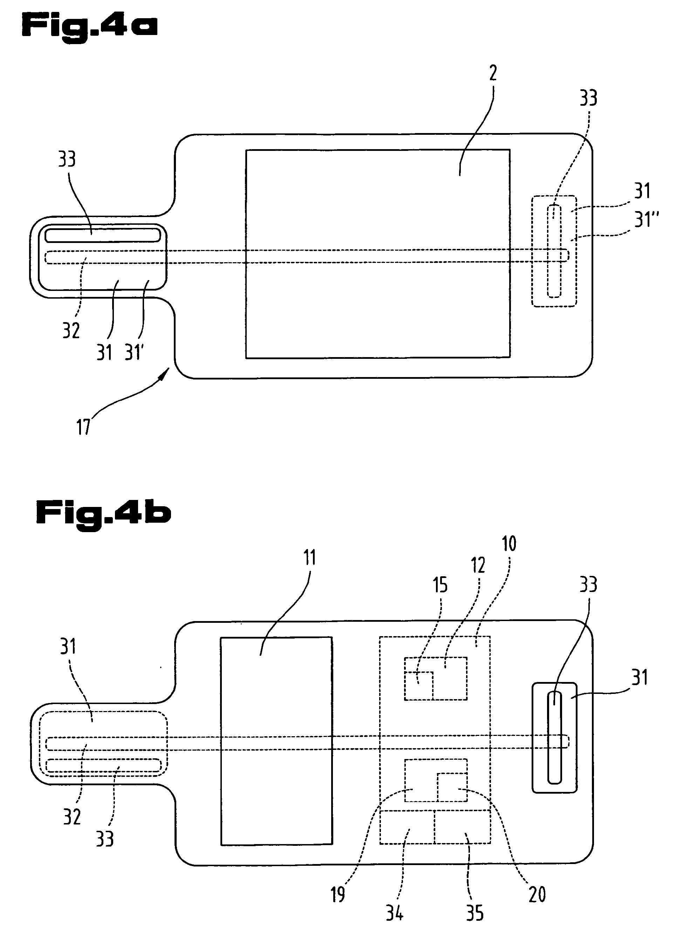

Fig. 4a und 4b zeigen eine weitere beispielhafte Ausbildung einer Authentifizierungsvorrichtung 17. Fig. 4a zeigt wiederum die Ansicht auf den Sensor 2, wobei diese Seite üblicherweise auf der Hautoberfläche aufliegt. Die Authentifizierungsvorrichtung 17 ist als Manschette die einen Körperteil umfasst ausgebildet und wird durch Verbinden der beiden Teile 31', 31" des Verschlusselements 31, auf diesem kraft- bzw. formschlüssig fixiert.

Das Verschlusselement 31 ist dabei derart ausgebildet, dass durch Verschluss bzw. in Eingriff bringen der beiden Teile 31', 31" des Verschlussmittels 31, ein sog. Trigger ausgelöst wird. Durch diesen Trigger wird der Authentifizierungsvorgang initiiert und ein biometrisches. Merkmal erfasst. Nach ermittelter Übereinstimmung mit, im Speicher 15 hinterlegten Referenzwerten, wird die Zuordnung zur Verriegelungsvorrichtung hergestellt und hernach kontinuierlich und/oder zeitdiskret ein unverwechselbarer Benutzercode übermittelt. Solange das Verschlusselement 31 geschlossen ist oder im Eingriff bleibt, ist keine weitere Erfassung eines biometrischen Merkmals erforderlich. Beim Lösen des Verschlusselements wird wiederum ein Trigger ausgelöst, der bspw. dazu verwendet werden kann, die Zuordnung zur Verriegelungsvorrichtung aufzuheben. Die Trigger auslösende Vorrichtung kann bspw. durch jede Art von Schalter oder Impulsgeber gebildet sein, der sowohl kontaktbehaftet, als auch kontaktlos arbeitet.

Da die Erfassung und Auswertung biometrischer Merkmale elektrische Energie benötigt, diese aber auf einer kompakten Authentifizierungsvorrichtung nur begrenzt zur Verfügung steht, wird gemäß dieser Ausbildung eine bedeutende Menge Energie eingespart, wodurch sich die Betriebsdauer der Authentifizierungsvorrichtung wesentlich verlängert.

Um zu verhindern, dass eine verriegelte bzw. verschlossene und erfolgreich authentifizierte Authentifizierungsvorrichtung 17 missbräuchlich verwendet wird, bspw. durch Auftrennen der Vorrichtung unter Erhalt des Verschlusses bzw. des Eingriffs des Verschlusselements 31, kann in der Authentifizierungsvorrichtung 17 ein Authentizitätsmerkmal 32 angeordnet sein. Dieses Authentizitätsmerkmal 32 ist derart ausgebildet, dass eine missbräuchliche Verwendung bzw. Manipulation der Authentifizierungsvorrichtung 17 verhindert wird bzw. als solche eindeutig erkennbar ist. Insbesondere wird eine erfolgte Zuordnung zu einer Verriegelungsvorrichtung bei einer erkannten Manipulation aufgehoben. Bevorzugt wird eine Ausbildung, bei der das Authentizitätsmerkmal auch ein Triggersignal auslösen kann.

Da weiters im Speicher 15 des Auswerte- und Vergleichsmoduls 10 Referenzdaten 14 hinterlegt sind, die einer missbräuchlichen Verwendung Raum geben, kann in einer Weiterbildung das Authentizitätsmerkmal 32 derart ausgebildet sein, dass ein Manipulationsversuch zur unwiederbringlichen Zerstörung dieser Referenzdaten führt.

Der Umfang des Körperteils, auf dem die Authentifizierungsvorrichtung angeordnet wird, kann ebenfalls als charakteristisches Merkmal betrachtet werden. Das Verschlusselement 31 weist bspw. eine Längenmessvorrichtung 33 auf, die bei geschlossenem Verschlusselement 31 den umschlossenen Umfang ermittelt. Stimmt der Umfang mit einem hinterlegten Umfang überein, kann dies für eine geringe Sicherheitsstufe bereits als Authentifikation ausreichen. Bspw. könnte diese Übereinstimmung aber auch als Trigger dienen und einen biometrischen Authentifizierungsvorgang auslösen. Die Längenmessvorrichtung 33 kann bspw. durch einen mechanisch beeinflussbaren, veränderlichen Widerstand gebildet sein, es sind aber auch optische Distanzerfassungsmittel einsetzbar. Dem kundigen Fachmann sind mehrere kompakte Vorrichtungen bzw. Verfahren zur Bestimmung einer Länge bekannt.

Eine Positionsortungseinrichtung 34 ermöglicht die Feststellung der Position der Authentifizierungsvorrichtung 17 durch eine Kontroll- bzw. Überwachungsstelle und/oder ermöglicht es der Authentifizierungsvorrichtung, die Position innerhalb eines Abschnitts selbsttätig zu bestimmen. Bevorzugt ist die Positionsortungseinrichtung 34 drahtlos ausgebildet bspw. durch GPS oder d-GPS.

Durch ein Netzwerk-Kommunikationsmodul 35, bevorzugt durch ein drahtloses Kommunikationsmodul, kann die Authentifizierungsvorrichtung 17 bspw. mit einer Kontroll- und/oder Überwachungsstelle Daten austauschen. Gemäß einer vorteilhaften Weiterbildung ist der Sensor 2 zur Erfassung von Vitalsignalen ausgebildet, wobei die erfassten Vitalsignale über die Kommunikationsverbindung des Netzwerk-Kommunikationsmodul 35 an die Kontroll- und/oder Überwachungsstelle übermittelt werden können. Diese ist somit in der Lage, jederzeit den Gesundheitszustand des Trägers zu überwachen. Insbesondere kann sichergestellt werden, dass eine Authentifikation nur von einer Person mit Vitalzeichen durchgeführt wird.

Die Ausführungsbeispiele zeigen mögliche Ausführungsvarianten der Sicherungsvorrichtung wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst.

Der Ordnung halber sei abschließend darauf hingewiesen, dass zum besseren Verständnis des Aufbaus der Sicherungsvorrichtung, diese bzw. deren Bestandteile teilweise unmaßstäblich und/oder vergrößert und/oder verkleinert dargestellt wurden.

### Bezugszeichenaufstellung

- 1: Sicherungsvorrichtung
- 2: Sensor
- 3: Quelle für elektromagnetische Strahlung
- 4: Detektor für elektromagnetische Strahlung
- 5: Körperteil

- 6: Unterarm
- 7: Hautoberflächenstruktur
- 8: Venenstruktur
- 10: Auswerte- und Vergleichsmodul

- 11: Energieversorgungseinrichtung
- 12: Recheneinheit
- 13: Vergleichsmodul
- 14: biometrische Referenzdaten
- 15: Speicher

- 16: Personenspezifischer Datensatz
- 17: Authentifizierungsvorrichtung
- 18: Verriegelungsvorrichtung
- 19: Kommunikationsmodul
- 20: Ver- und/oder Entschlüsselungsmodul

- 21: Kommunikationsverbindung
- 22: Auswertemodul
- 23: Antriebsmittel
- 24: Sicherungselement
- 25: Oberarm

- 26: Halsmikrofon
- 27: Augenschutz
- 28: Halteriemen
- 29: Kopfbedeckung / Helm
- 30: Tragegurt

- 31: Verschlussmittel
- 32: Authentizitätsmerkmal
- 33: Längenmessvorrichtung
- 34: Positionsortungseinrichtung
- 35: Netzwerk-Kommunikationsmodul

- 36: Fußgelenk

## Patentansprüche

1. Sicherungsvorrichtung (1) umfassend zumindest eine Authentifizierungsverrichtung (17) und eine Verriegelungsvorrichtung (18) wobei die Authentifizierungsvorrichtung (17) zumindest einen Sensor (2) und ein Auswerte- und Vergleichsmodul (10) umfasst und wobei zwischen der Verriegelungsvorrichtung (18) und der Authentifizierungsvorrichtung (17) eine Kommunikationsverbindung (21) besteht, **dadurch gekennzeichnet, dass** der Sensor (2) als elastisch rückstellbarer Dünnfilmsensor zur Erfassung biometrischer Daten bzw. spektraler Eigenschaften der Haut sowie darunter liegender Gewebeschichten ausgebildet ist, und dass die durch das Nahfeld der Haut des Benutzers gebildete Kommunikationsverbindung (21) zu sicheren, drahtlosen Übertragung eines, von der Authentifizierungsvorrichtung (17) ermittelten, unverwechselbaren Benutzercodes ausgebildet ist und in ihrem Wirkbereich auf einen Nahbereich, insbesondere weniger als 50cm, beschränkt ist, und dass die Verriegelungsvorrichtung (18) bei Übereinstimmung des Benutzercodes mit einem, der Verriegelungsvorrichtung zugeordneten, Identifikationscode deaktiviert ist.

2. Sicherungsvorrichtung nach Anspruch 1, **dadurch gekennzeichnet, dass** der Sensor (2) aus zumindest einem Material der Gruppe umfassend organische halbleitende Materialien, anorganische halbleitende Materialien, Nanopartikel, gebildet ist.

3. Sicherungsvorrichtung nach einem der Ansprüche 1 oder 2, **dadurch gekennzeichnet, dass** der Sensor (2) zumindest eine Quelle (3) und zumindest einen Quantendetektor (4) umfasst.

4. Sicherungsvorrichtung nach Anspruch 3, **dadurch gekennzeichnet, dass** die Quelle (3) zur Abgabe von elektromagnetischer Strahlung mit einer Wellenlänge im Bereich 350nm bis 780nm ausgebildet ist.

5. Sicherungsvorrichtung nach Anspruch 3 oder 4, **dadurch gekennzeichnet, dass** die Quelle (3) zur Abgabe von elektromagnetischer Strahlung mit einer Wellenlänge im Bereich 750nm bis 1,4µm ausgebildet ist.

6. Sicherungsvorrichtung nach einem der Ansprüche 1 bis 5, **dadurch gekennzeichnet, dass** der Quantendetektor (4) als Anordnung einer Mehrzahl fotosensitiver Elemente ausgebildet ist.

7. Sicherungsvorrichtung nach einem der Ansprüche 1 bis 6, **dadurch gekennzeichnet, dass** der Sensor (2) als Folie, insbesondere als Klebefolie, ausgebildet ist.

8. Sicherungsvorrichtung nach einem der Ansprüche 1 bis 7, **dadurch gekennzeichnet, dass** die Authentifizierungsvorrichtung (17) eine Energieversorgungseinrichtung (11) umfasst, wobei die Energieversorgungseinrichtung (11) gebildet ist aus der Gruppe umfassend ein elektrochemisches Element, einen kapazitiven Energiespeicher oder eine Solarzelle.

9. Sicherungsvorrichtung nach einem der Ansprüche 1 bis 8, **dadurch gekennzeichnet, dass** das Auswerte- und Vergleichsmodul (10) und der Sensor (2) integriert angeordnet sind.

10. Sicherungsvorrichtung nach einem der Ansprüche 1 bis 9, **dadurch gekennzeichnet, dass** das Auswerte- und Vergleichsmodul (10) einen Speicher (15) umfasst, wobei im Speicher (15) biometrische Referenzdaten (14) hinterlegt sind.

11. Sicherungsvorrichtung nach Anspruch 10, **dadurch gekennzeichnet, dass** das Auswerte- und Vergleichsmodul (10) eine Recheneinheit (12) umfasst, wobei die Recheneinheit (12) zur Auswertung, der vom Sensor (2) erfassten biometrischen Daten und zum Vergleich der ausgewerteten biometrischen Daten mit im Speicher (15) hinterlegten Referenzdaten (14) ausgebildet ist.

12. Sicherungsvorrichtung nach einem der Ansprüche 1 bis 11, **dadurch gekennzeichnet, dass** die Authentifizierungsvorrichtung (17) einen Energiesparbetriebszustand aufweist, in dem der Energieverbrauch kleiner 500µW ist.

13. Sicherungsvorrichtung nach einem der Ansprüche 1 bis 12, **dadurch gekennzeichnet, dass** die Authentifizierungsvorrichtung (17) ein drahtloses Positionsortungssystem (34) aufweist.

14. Sicherungsvorrichtung nach einem der Ansprüche 1 bis 13, **dadurch gekennzeichnet, dass** die Authentifizierungsvorrichtung (17) ein Verschlusselement (31) aufweist, das zur Abgabe eines Triggersignals ausgebildet ist.

15. Sicherungsvorrichtung nach einem der Ansprüche 1 bis 14, **dadurch gekennzeichnet, dass** die Authentifizierungsvorrichtung (17) ein Authentizitätsmerkmal (32) aufweist.

16. Sicherungsvorrichtung nach einem der Ansprüche 1 bis 15, **dadurch gekennzeichnet, dass** die Authentifizierungsvorrichtung (17) eine Längenmessvorrichtung (33) aufweist.

17. Sicherungsvorrichtung nach einem der Ansprüche 1 bis 16, **dadurch gekennzeichnet, dass** die Verriegelungsvorrichtung (18) zur Herstellung einer zeitbegrenzten datentechnischen Zuordnung zur Authentifizierungsvorrichtung (17) ausgebildet ist.

18. Sicherungsvorrichtung nach einem der Ansprüche 1 bis 17, **dadurch gekennzeichnet, dass** die Verriegelungsvorrichtung (17) ein Sicherungselement (24), insbesondere einen Sicherungsbolzen, und ein Antriebsmittel (23) umfasst.

19. Sicherungsvorrichtung nach einem der Ansprüche 1 bis 18, **dadurch gekennzeichnet, dass** die Verriegelungsvorrichtung (17) durch einen elektronischen Zündgeber gebildet ist.

20. Sicherungsvorrichtung nach einem der Ansprüche 1 bis 19, **dadurch gekennzeichnet, dass** die Verriegelungsvorrichtung (17) eine Statusanzeige aufweist.

## Claims

1. Security device (1) comprising at least one authentication device (17) and a locking device (18), which authentication device (17) comprises at least one sensor (2) and an evaluation and comparison module (10), and a communication link (21) exists between the locking device (18) and the authentication device (17), **characterized in that** the sensor (2) is provided in the form of an elastically reboundable thin-film sensor for detecting biometric data or spectral properties of the skin and layers of tissue lying underneath, and the communication link (21) is programmed to effect a secure, wireless transmission of a unique user code determined by the authentication device (17) and is limited in terms of its operating range to a close-up range, in particular less than 50cm, and the locking device (18) is deactivated if the user code matches an identification code assigned to the locking device.

2. Security device according to claim 1, **characterized in that** the sensor (2) is made from at least one material from the group comprising organic semiconductor materials, inorganic semiconductor materials, nano-particles.

3. Security device according to one of claims 1 or 2, **characterized in that** the sensor (2) comprises at least one source (3) and at least one quantum detector (4).

4. Security device according to claim 3, **characterized in that** the source (3) is designed to emit electromagnetic radiation with a wavelength in the range of 350nm to 780nm.

5. Security device according to claim 3 or 4, **characterized in that** the source (3) is designed to emit electromagnetic radiation with a wavelength in the range of 750nm to 1.4µm.

6. Security device according to one of claims 1 to 5, **characterized in that** the quantum detector (4) is provided in the form of an array of several photosensitive elements.

7. Security device according to one of claims 1 to 6, **characterized in that** the sensor (2) is provided in the form of a film, in particular adhesive film.

8. Security device according to one of claims 1 to 7, **characterized in that** the authentication device (17) has a power supply unit (11) and the power supply unit (11) is selected from the group comprising an electro-chemical element, a capacitive element or a solar cell.

9. Security device according to one of claims 1 to 8, **characterized in that** the evaluation and comparison module (10) and the sensor (2) are integrated.

10. Security device according to one of claims 1 to 9, **characterized in that** the evaluation and comparison module (10) has a memory (15) and biometric reference data (14) is stored in the memory (15).

11. Security device according to claim 10, **characterized in that** the evaluation and comparison module (10) has a computer unit (12) and the computer unit (12) is programmed to evaluate the biometric data detected by the sensor (2) and compare the evaluated biometric data with reference data (14) stored in the memory (15).

12. Security device according to one of claims 1 to 11, **characterized in that** the authentication device (17) has a power save operating mode in which energy consumption is less than 500µW.

13. Security device according to one of claims 1 to 12, **characterized in that** the authentication device (17) has a wireless position sorting system (34).

14. Security device according to one of claims 1 to 13, **characterized in that** the authentication device (17) has a closure element (31) which is designed to emit a trigger signal.

15. Security device according to one of claims 1 to 14, **characterized in that** the authentication device (17) has an authenticity feature (32).

16. Security device according to one of claims 1 to 15, **characterized in that** the authentication device (17) has a length measuring device (33).

17. Security device according to one of claims 1 to 16, **characterized in that** the locking device (18) is designed to set up a data assignment to the authentication device (17) for a limited time.

18. Security device according to one of claims 1 to 17, **characterized in that** the locking device (17) comprises a lock element (24), in particular a lock bolt, and a drive means (23).

19. Security device according to one of claims 1 to 18, **characterized in that** the locking device (17) is provided in the form of an electronic firing system.

20. Security device according to one of claims 1 to 19, **characterized in that** the locking device (17) has a status display.

## Revendications

1. Dispositif de sécurité (1) comprenant au moins un dispositif d'authentification (17) et un dispositif de verrouillage (18), où le dispositif d'authentification (17) comprend au moins un capteur (2) et un module d'évaluation et de comparaison (10), et où existe entre le dispositif de verrouillage (18) et le dispositif d'authentification (17) une liaison de communication (21), **caractérisé en ce que** le capteur (2) est réalisé comme capteur à film mince à rappel élastique pour la détection de données biométriques respectivement de propriétés spectrales de la peau ainsi que de couches de tissu situées en dessous, et **en ce que** la liaison de communication (21) formée par le champ proche de la peau de l'utilisateur est réalisée pour la transmission sûre sans fil d'un code d'utilisateur irréversible, déterminée par le dispositif d'authentification (17) et est limitée dans sa zone d'action à une zone proche, en particulier inférieure à 50 cm, et **en ce que** le dispositif de verrouillage (18), lors de la coïncidence du code d'utilisateur avec un code d'identification associé au dispositif de verrouillage, est désactivé.

2. Dispositif de sécurité selon la revendication 1, **caractérisé en ce que** le capteur (2) est formé par au moins un matériau du groupe comprenant des matériaux semi-conducteurs organiques, des matériaux semi-conducteurs inorganiques, des nanoparticules.

3. Dispositif de sécurité selon l'une des revendications 1 ou 2, **caractérisé en ce que** le capteur (2) comprend au moins une source (3) et au moins un détecteur quantique (4).

4. Dispositif de sécurité selon la revendication 3, **caractérisé en ce que** la source (3) est réalisée pour l'émission d'un rayonnement électromagnétique d'une longueur d'onde dans la plage de 350 nm à 780 nm.

5. Dispositif de sécurité selon la revendication 3 ou 4, **caractérisé en ce que** la source (3) est réalisée pour l'émission d'un rayonnement électromagnétique d'une longueur d'onde dans la plage de 750 nm à 1,4 µm.

6. Dispositif de sécurité selon l'une des revendications 1 à 5, **caractérisé en ce que** le détecteur quantique (4) est réalisé comme agencement d'une pluralité d'éléments photosensibles.

7. Dispositif de sécurité selon l'une des revendications 1 à 6, **caractérisé en ce que** le capteur (2) est réalisé comme feuille, en particulier comme feuille collante.

8. Dispositif de sécurité selon l'une des revendications 1 à 7, **caractérisé en ce que** le dispositif d'authentification (17) comprend une installation d'alimentation en énergie (11), où l'installation d'alimentation en énergie (11) est formée à partir du groupe comprenant un élément électrochimique, un accumulateur d'énergie capacitif ou une pile solaire.

9. Dispositif de sécurité selon l'une des revendications 1 à 8, **caractérisé en ce que** le module d'évaluation et de comparaison (10) et le capteur (2) sont intégrés.

10. Dispositif de sécurité selon l'une des revendications 1 à 9, **caractérisé en ce que** le module d'évaluation et de comparaison (10) comprend une mémoire (15), et dans la mémoire (15), des données de référence biométriques (14) sont stockées.

11. Dispositif de sécurité selon la revendication 10, **caractérisé en ce que** le module d'évaluation et de comparaison (10) comprend une unité de calcul (12), l'unité de calcul (12) étant réalisée pour l'évaluation des données biométriques détectées par le capteur (2) et pour la comparaison des données biométriques évaluées avec des données de référence (14) stockées dans la mémoire (15).

12. Dispositif de sécurité selon l'une des revendications 1 à 11, **caractérisé en ce que** le dispositif d'authentification (17) présente un état de fonctionnement à économie d'énergie, où la consommation en énergie est inférieure à 500µ W.

13. Dispositif de sécurité selon l'une des revendications 1 à 12, **caractérisé en ce que** le dispositif d'authentification (17) présente un système de triage de position sans fil (34).

14. Dispositif de sécurité selon l'une des revendications 1 à 13, **caractérisé en ce que** le dispositif d'authentification (17) présente un élément de fermeture (31) qui est réalisé pour l'émission d'un signal de déclenchement.

15. Dispositif de sécurité selon l'une des revendications 1 à 14, **caractérisé en ce que** le dispositif d'authentification (17) présente une caractéristique d'authenticité (32).

16. Dispositif de sécurité selon l'une des revendications 1 à 15, **caractérisé en ce que** le dispositif d'authentification (17) présente un dispositif de mesure de longueur (33).

17. Dispositif de sécurité selon l'une des revendications 1 à 16, **caractérisé en ce que** le dispositif de verrouillage (18) est réalisé pour l'établissement d'une attribution informatique limitée dans le temps au dispositif d'authentification (17).

18. Dispositif de sécurité selon l'une des revendications 1 à 17, **caractérisé en ce que** le dispositif de verrouillage (17) comprend un élément de sécurité (24), en particulier un boulon de sécurité ainsi qu'un moyen d'entraînement (23).

19. Dispositif de sécurité selon l'une des revendications 1 à 18, **caractérisé en ce que** le dispositif de verrouillage (17) est formé par un capteur d'allumage électronique.

20. Dispositif de sécurité selon l'une des revendications 1 à 19, **caractérisé en ce que** le dispositif de verrouillage (17) présente un affichage de statut.
